# EUROPEAN PATENT SPECIFICATION

(11) **EP 1 917 047 B1**
(45) Date of publication and mention of the grant of the patent: **04.09.2019**
(21) Application number: 06764975.6
(22) Date of filing: 14.07.2006
(51) Int. Cl.: A61L 15/60, A61L 26/00, C08L 101/02, A61P 17/02

(54) **TREATMENT OF CHRONIC ULCEROUS SKIN LESIONS**
BEHANDLUNG VON CHRONISCHEN GESCHWÜRIGEN HAUTVERLETZUNGEN
TRAITEMENT DE LESIONS CUTANEES ULCEREUSES CHRONIQUES

(30) Priority: 14.07.2005 GB 0514526; 14.07.2005 US 699449 P; 03.11.2005 GB 0522530; 17.05.2006 GB 0609827; 17.05.2006 GB 0609828
(43) Date of publication of application: 07.05.2008
(73) Proprietor: First Water Limited, Marlborough, Wiltshire SN8 2RB (GB)
(72) Inventor: MUNRO, Hugh Semple, Chipping Campden Warwickshire GL55 6QT (GB)
(74) Representative: Haseltine Lake Kempner LLP
(86) International application number: PCT/GB2006/002632
(87) International publication number: WO 2007/007115

(56) References cited:
- EP-A- 1 026 219
- WO-A-00/07636
- WO-A-00/07638
- WO-A-00/65143
- WO-A-01/96422
- WO-A-91/15250
- WO-A-03/077964
- US-A- 4 909 244
- US-A1- 2001 055 608
- US-B1- 6 306 419
- DEGIM Z ET AL: "An investigation on skin wound healing in mice with a taurine-chitosan gel formulation" AMINO ACIDS (VIENNA), vol. 22, no. 2, 2002, pages 187-198, XP002423538 ISSN: 0939-4451
- HAMPTON S: "A SMALL STUDY IN HEALING RATES AND SYMPTOM CONTROL USING A NEW SHEET HYDROGEL DRESSING" JOURNAL OF WOUND CARE, MACMILLAN, LONDON, GB, vol. 13, no. 7, July 2004 (2004-07), pages 297-300, XP009078408 ISSN: 0969-0700
- VACHON DAVID J ET AL: "Novel sulfonated hydrogel composite with the ability to inhibit proteases and bacterial growth." JOURNAL OF BIOMEDICAL MATERIALS RESEARCH. PART A JAN 2006, vol. 76, no. 1, January 2006 (2006-01), pages 35-43, XP002423539 ISSN: 1549-3296

## Description

### Field of the Invention

The present invention relates to the treatment of chronic ulcerous skin lesions, in humans and other mammals, particularly humans, and more particularly to the use of a hydrogel composition or dressing for treatment of chronic ulcerous skin lesions, to promote their healing.

The present invention introduces the "Pro-Ionic™" treatment of wounds, which is a novel concept in which a hydrogel dressing in contact with the wound provides in use a controlled-moisture environment for the wound and selective uptake of proteins and ions from the wound, to stimulate and/or maintain the wound healing process.

Without wishing to be bound by theory, the hydrogel dressing is believed to mimic the natural extracellular matrix of a normal healing wound, and in particular certain sulphonated proteoglycans of the extracellular matrix such as heparin, using a moist wound healing environment where, in contrast to prior methods, the water levels are controlled to avoid the disadvantages of too much or too little moisture. In the case of chronic wounds, the dressing suppresses the processes that lead to chronic failure of the wound to heal and stimulates and/or maintains the normal healing process.

It has also been found that, in the case of acute wounds, the dressing suppresses any tendency towards chronic failure to heal, and stimulates and /or maintains the normal healing process.

The hydrogel used is a certain type of hydrous hydrophilic (ionic) polymer, described in more detail below. The ions covalently linked to the polymer molecule are anions; the cations are present as counterions (mono- metal ions, i.e. sodium counterions, alone or in combination with a potassium counterions). The polymer in the hydrogel, including its associated water and ions, provides one or more, for example simultaneously any two or more, of the following beneficial effects on the wound, without the need for other bioactive agents, namely: (1) beneficial antimicrobial action, (2) beneficial wound debridement, (3) beneficial skin conditioning, (4) beneficial pain relief, and (5) in combination, beneficial suppression of the processes which lead to, and/or maintain, a chronic wound with beneficial wound bed stimulation and/or maintenance of the healing process. Preferably, the beneficial effects on the wound include beneficial antimicrobial action and simultaneously one or more, more preferably two or more, more preferably three or all, of effects (2) to (5).

The present disclosure also relates to rapid killing of microbes in the context of the treatment of wounds.

### Background of the Invention

### Lesion Healing Process

The normal process of healing of a skin lesion (wound) typically proceeds via four distinct sequential stages or phases, namely haemostasis, inflammation, proliferation and maturation.

Haemostasis is the vascular response stage, occurring immediately after the insult is suffered, and normally lasts for up to about three days in humans. The wound may bleed initially, and the blood then clots.

Inflammation normally arises about one after the insult, and typically continues until about six days after the insult. Inflammation involves one or more of redness, heat, swelling and pain. The wound starts to exude fluid, which serves to remove debris, and proteases are released into the wound area. White blood cells and macrophages begin to congregate in the lesion zone, the former to clear debris and the latter for phagocytosis and to release growth factors to stimulate fibroblasts. During this phase the extracellular matrix is constructed.

Proliferation normally arises about four days after the insult, and typically continues until about 21 days after the insult, and involves the gradual formation of granulation tissue to fill the lesion zone. The redness, heat, swelling and pain gradually subside during this phase. For these reasons, granulation and contracture are sometimes identified as sub-phases within the proliferation phase. During proliferation, the macrophages stimulate vascular endothelial growth factor (VEGF) to stimulate new blood vessel growth, and the concentration of fibroblasts increase, producing collagen for the new tissues.

The maturation phase normally arises about 21 days after the insult, and typically continues for several weeks, months or even years thereafter. Maturation involves contraction of the wound, growth of new epithelial tissue covering the wound, and possibly scar formation. During this phase myofibroblasts develop from the fibroblasts and the collagen fibres gradually mature and become relatively more organised.

Generally, different parts of a wound heal at different rates, so that it is common for some parts of a normal wound to be at a more advanced stage of healing than others.

The above timescale of a normal wound is provided for general illustration only, and is not definitive for all normal wound healing. The present invention is not limited by any requirement that the normal wound healing process must follow any particular pathway or timescale.

### Chronic Ulcerous Skin Lesions

Chronic skin lesions arise when a skin wound generally fails to follow an appropriate timely healing process to achieve the normal sustained and stable anatomic and functional integrity of the healed tissue. Generally speaking, a skin lesion which has failed to make at least substantial progress towards healing within a period of at least about three months, or which has become stable in a partially healed state for more than about three months, could be categorised as chronic, although even this general guide is not an absolute marker as the age and fitness of the patient, as well as other factors such as diseases or disorders suffered by the patient (for example, circulatory disorders), can significantly lengthen the normal healing process. A skin lesion which is unhealed after at least about six months can be categorised as chronic.

A chronic skin lesion is ulcerous where it involves focal loss of the epidermis and at least part of the dermis.

Malignant or pre-malignant chronic ulcerous skin lesions may arise in connection with a primary cancer of the skin, or with a metastasis to the skin from a local tumour or from a tumour in a distant site. They may be draining or non-draining. They may, for example, take the form of a cavity, an open area on the surface of the skin, skin nodules, or a nodular growth extending from the surface of the skin.

Benign chronic ulcerous skin lesions are not associated with cancer, and include venous leg ulcers, venous foot ulcers, arterial leg ulcers, arterial foot ulcers, decubitus ulcers (e.g. pressure sores, bedsores), post-surgical ulcerous lesions and chronic burn lesions. They may, for example, take the form of a cavity, an open area on the surface of the skin, skin nodules, or a nodular growth extending from the surface of the skin. Typically, they comprise an open granulating area on the surface of the skin.

Chronic ulcerous skin lesions are usually accompanied by other chronic symptoms apart from the failure of the normal healing process. Typical accompanying chronic symptoms include one or more of pain, exudation, malodour, excoriation, spreading of the wound, tissue necrosis, irritation and hyperkeratosis. Such symptoms can be extremely debilitating and embarrassing for patients, and can seriously harm the patient's quality of life. In severe cases, they can require amputation of limbs or even death.

Chronic ulcerous skin lesions can also be categorised according to their exudation. General categorisation is into the three categories "high exudation", "medium exudation" and "low exudation". Exudate management is a particularly difficult task for the caring professional attending to the patient. A balance needs to be struck between the desire to remove exudate to maintain the patient's quality of life at as high a level as possible, and maintenance of an appropriate level of fluid to prevent the lesion becoming too dry or too wet.

### Prior Art Treatments

WO-A-00/07638 discloses bioadhesive hydrogel compositions and their use in wound dressings. The polymer composition is stated to preferably comprise also a non-hydrophilic (hydrophobic) polymer, and may comprise a specifically antimicrobial agent such as citric acid or stannous chloride. No information is given as to any effects of the hydrogel compositions on the microbe populations of wounds, for example human skin wounds. More generally, there is no teaching that the polymer *per se* in the hydrogel, including its associated water and ions, provides any combination of the beneficial effects on the wound mentioned as (1) to (5) above, without the need for other bioactive agents.

It is known to apply dressings to chronic skin lesions, with the aim of promoting their healing. Examples of such prior art dressings for chronic ulcerous skin lesions include Aquacel™ (ConvaTec) (http://www.dressings.org/Dressings/aquacel.html), Intrasite™ (Smith & Nephew) (http://www.dressings.org/Dressings/intrasit.gel.html) and Avance (Medlock Medical) (http://www.medlockmedical.com/woundcare/avance.htm) .

Generally speaking, and without commenting specifically on the particular examples given above, prior art dressings for chronic ulcerous skin lesions suffer from a variety of problems. For example, they can cause maceration of peri-wound areas, they can absorb wound exudate only partially, they can cause contact dermatitis, varicose eczema or skin stripping (e.g. due to aggressive or allergenic adhesive materials). Furthermore, even in cases where the prior art dressings for chronic skin lesions contribute to successful healing, scarring of the healed wound and poor quality of healed tissue can often be found.

The prior art dressings for chronic ulcerous skin lesions can also be slow and difficult to apply and change, and require frequent changing. Many patients experience considerable - sometimes unbearable - pain associated with changing of the dressing, over and above the often considerable general pain associated with the lesion itself. The use of opiate painkillers to deal with this pain can lead to opiate dependency and addiction.

Prior art dressings that require frequent changing cause a significant increase in costs to healthcare services and providers, as a nurse or other healthcare professional needs to attend the patient correspondingly more often. In addition, the material costs of the dressings clearly are higher because of the frequent application of fresh dressings.

In an article entitled "A small study in healing rates and symptom control using a new sheet hydrogel dressing" in Journal of Wound Care, July 2004, 13(7), and in a poster presentation at the Tissue Viability Society (TVS) Conference in Torquay, UK, in April 2003, available on http://www.activahealthcare.co.uk/pdf/cs-actiformcool2.pdf, Sylvie Hampton described a study into the effects of a sheet hydrogel dressing on chronic leg and foot ulcers of at least six months duration (average 9 months to two years) in 16 human patients. The pre-treatment ulcers of almost all of the patients were either high exudation or medium exudation. The sheet hydrogel dressing was supplied by Activa Healthcare of Burton-upon-Trent, UK (tel: + 44 8450 606 707; web: www.activahealthcare.co.uk) under the name ActiFormCool™.

The results published by Sylvie Hampton showed the potential for substantial advantages deriving from the use of ActiFormCool™ as a dressing in the treatment of chronic leg and foot ulcers. However, neither the Journal of Wound Care article nor the poster presentation mentioned above disclosed the underlying nature of the therapeutic effect or the nature of any active component of the composition of ActiFormCool™. No information was given as to any effects of the hydrogel compositions on the microbe populations of wounds. More generally, there was no teaching that the polymer *per se* in the hydrogel, including its associated water and ions, provides any combination of the beneficial effects on the wound mentioned as (1) to (5) above, without the need for other bioactive agents.

We have now found a relationship between the presence and number of multiple pendant sulphonyl groups and optionally also multiple pendant carboxylic groups on each polymer molecule of a hydrogel wound dressing material and the therapeutic effects of the material, including without limitation a rapid antimicrobial action of the material. This finding for the first time makes effective treatment available to a wider class of patients having chronic ulcerous skin lesions and in particular chronic leg and foot ulcers that are refractory to prior art treatments. Patients who have reactions to certain classes of antibiotics, painkillers or other bioactive agents conventionally used in, or in conjunction with, wound dressings, or who are addicted to or dependent on opiate or other powerful painkillers conventionally used in conjunction with wound care, will be treatable using the present invention - in which the use of other bioactive agents such as antibiotics or painkillers can be avoided - whereas previous treatment protocols were restricted by the need to avoid the problematic antibiotics, painkillers or other bioactive agents. Therefore, the novel findings constitute and make available a novel therapeutic application.

By "pendant sulphonyl groups" we mean sulphonyl (-SO₂-) containing groups, most particularly sulpho (-SO₂-OH) groups salt form or organic groups which include sulpho (-SO₂-OH) groups in salt form, which extend from the carbon atom containing chain ("carbon chain") of the polymer molecule and are covalently linked (pendant) to the carbon chain. Where the sulphonyl containing group is an organic group which includes the sulphonyl moiety, e.g. in a sulpho (-SO₂-OH) group in salt form, the sulphonyl moiety is preferably located at or near the terminal free end of the organic group, i.e. the end distant from the carbon chain of the polymer molecule.

Some or all of the sulpho groups (-SO₂-OH) groups in salt form may, if desired, be O-linked to the carbon chain of the polymer molecule, for example as organic sulphate groups.

The sulpho groups in salt form are an alkali metal salt of the acid form (-SO₂-OH). The salt form will comprise sodium ions, either alone or in combination with the potassium salt form. A combination of sodium and potassium counterions can be particularly suitable.

The organic sulphonyl containing groups or some of them may contain a carboxylate or carboxamido linkage unit. The polarity of these species, in conjunction with the sulphonyl groups, seems to play a part in achieving the desirable effects underlying the present invention. It is preferred that the carboxylate or carboxamido linkage unit, when present, is closer to the carbon chain of the polymer than the sulphonyl moiety.

By "pendant carboxylate groups" we mean carboxylate (-CO₂-) containing groups, most particularly carboxylic acid (-CO₂H) groups in acid or salt form or organic groups which include carboxylic acid (-CO₂H) groups in acid or salt form, which extend from the carbon atom containing chain ("carbon chain") of the polymer molecule and are covalently linked (pendant) to the carbon chain. Where the carboxylate containing group is an organic group which includes the carboxylate moiety, the carboxylate moiety is preferably located at or near the terminal free end of the organic group, i.e. the end distant from the carbon chain of the polymer molecule.

Where carboxylic acid groups or some of them are present in salt form, the salt form may suitably be an alkali or alkaline earth or other multivalent (e.g. transition) metal or ammonium or organo-ammonium salt of the acid form (-CO₂H). For example, the salt form may be the sodium, potassium, lithium, caesium, calcium, magnesium, zinc or ammonium salt or combinations thereof. Preferably the salt form will comprise sodium ions, either alone or in combination with one or more other salt forms such as, for example, potassium, magnesium, zinc or calcium. A combination of sodium and potassium counterions can be particularly suitable. Where a combination of counterions is present in the hydrogel, any multivalent counterion (e.g. one or more of magnesium, zinc, calcium) is suitably present in a total molar proportion of up to about 5 mol% relative to the sodium ions.

Sulphonated hydrophilic polymers are known to have antagonist activity towards fibroblast growth factor-2 (FGF-2), and consequently their use as potential inhibitors of FGF-2-induced endothelial cell proliferation in angiogenesis and tumour growth has been proposed (S Liekens et al, Molecular Pharmacology, 56, pages 204 to 213 (1999)). In view of this, our novel finding that the polymers can promote healing of wounds, when applied as a hydrous hydrophilic ionic hydrogel in contact with a wound, is surprising and not obvious. Our current understanding of the mode of action of the invention is explained below, and is compatible with the reported FGF-2-antagonistic activity of the (un-crosslinked) polymers in solution.

### Brief Description of the Invention

According to an aspect of the present invention, there is provided a topical hydrogel composition comprising a hydrophilic polymer carrying multiple pendant sulphonyl groups on each polymer molecule, for use in the treatment of a wound, for example a chronic skin lesion, in a human or non-human mammal, particularly a human,
wherein the sulfonyl groups contain sulpho groups in salt form,
wherein the salt form is sodium alone or a combination of sodium and potassium counterions, and the molar ratio of sodium ions to potassium ions in the hydrogel composition is in the range of about 100:0 to 100:10.

Herein is also disclosed, a method of treating a chronic ulcerous skin lesion, in a human or non-human mammal, particularly a human, comprising contacting the wound for an effective period of time with a topical hydrogel composition comprising a hydrophilic polymer carrying multiple pendant sulphonyl groups on each polymer molecule.

Herein is also disclosed, a method of treating a chronic ulcerous skin lesion, in a human or non-human mammal, particularly a human, comprising contacting the wound for an effective period of time with a topical hydrogel composition comprising a hydrophilic polymer carrying multiple pendant sulphonyl groups and multiple pendant carboxylic groups on each polymer molecule

The hydrogel composition comprises a polymer matrix holding a liquid (normally aqueous) phase retained within the hydrogel. The polymer matrix is preferably cross-linked. The degree of cross-linking may be varied as desired. The polymeric matrix preferably consists of a cross-linked hydrophilic polymer. The liquid phase may, if desired, incorporate one or more other bioactive agents (e.g. particularly agents soluble or miscible in the liquid held within the polymer matrix of the hydrogel) to assist the healing process of the chronic skin lesion, or may be free or substantially free of such bioactive agents. It is a preferred feature of the present invention, however, that the hydrogel composition *per se* can be effective for the treatment, without the need for other bioactive agents.

The hydrogel composition is preferably used in sheet form. The hydrogel composition is preferably prepared in sheet form by polymerisation of a laid down layer of a liquid pre-gel mixture of polymerisable components, which are then cured to provide the polymerised mass. Preferably all or substantially all of the desired components of the hydrogel composition, including any water, are present in the pre-gel, and that no or substantially no drying or other adjustments are required after polymerisation (apart from minor conventional conditioning).

The hydrogel composition is preferably a constituent of a dressing for the lesion (wound).

The contacting of the wound with the hydrogel composition comprising a hydrophilic polymer carrying multiple pendant sulphonyl groups, optionally with multiple pendant carboxylic groups, on each polymer molecule preferably takes place for a period of time or for a sequence of time periods to promote healing, preferably with simultaneous reduction in one or more of pain, exudation, malodour, excoriation, spreading of the wound, tissue necrosis, irritation and hyperkeratosis.

The effective amount of pendant sulphonyl groups, optionally with multiple pendant carboxylic groups, in the hydrogel, for treating the wound will vary from subject to subject, but generally speaking the effective amount is as described in more detail below, in the section headed "Detailed Description of the Invention; The Hydrogel, Dressing and Treatment". Adjustments to the sulphonyl and optional carboxylic groups to suit individual subjects will be within the capacity of one skilled in the art, following simple experimental procedures.

The effective period of time will vary from subject to subject, but generally speaking an effective period of time will be up to about six weeks, for example between about 3 days and 6 weeks, depending on the seriousness of the wound. Regular changes of the dressing will be required, particularly with more serious and exuding wounds. The time between changes of dressing will generally be in the range of about 2 to about 7 days, preferably about 3 to about 7 days. The hydrogel composition used in the present invention seems to require fewer changes per week on average, than prior conventional dressings used for the treatment of chronic ulcerous skin lesions. For example, a study of 20 patients having chronic leg and foot ulcers showed that the prior art dressings required on average 3.00 changes per week, whereas the dressing according to the present invention required on average 1.75 changes per week. This is highly advantageous, both in terms of cost and manpower demands on health services and in terms of the pain and inconvenience to patients.

Herein is also disclosed, the use of a hydrogel composition comprising a hydrophilic polymer carrying multiple pendant sulphonyl groups, optionally with multiple pendant carboxylic groups, on each polymer molecule, in the preparation of a topical medicament for the treatment of a chronic ulcerous skin lesion, in a human or non-human mammal, particularly a human

Herein is also disclosed, a method of rapidly killing microbes which comprises contacting the microbes for an effective period of time with a hydrogel composition comprising a hydrophilic polymer carrying multiple pendant sulphonyl groups on each polymer molecule.

Herein is also disclosed, a method of rapidly killing microbes which comprises contacting the microbes for an effective period of time with a hydrogel composition comprising a hydrophilic polymer carrying multiple pendant sulphonyl groups and multiple pendant carboxylic groups on each polymer molecule.

The expression "rapid killing" used herein refers in particular to a speed of killing which, when tested *in vitro,* causes at least about a 500-fold reduction in microbe concentration in an aqueous microbe-containing medium (colony forming units (cfu's) per ml) in about 48 hours. More especially, the said rate of reduction in microbe concentration may be at least about 750-fold, for example at least about 1000-fold. In some cases, the said rate of reduction in microbe concentration may be at least about 5000-fold, for example at least about 10000-fold, for example at least about 20000-fold.

Without wishing to be bound by theory, it is believed that the present disclosure works at least in part through a mechanism of rapid nutrient uptake from the aqueous microbe-containing medium into the hydrogel, whereby the aqueous medium is depleted of microbe-sustaining nutrients, leading to rapid death of the microbes.

Herein is also disclosed, a method of denutrifying an aqueous microbe-containing liquid medium which comprises contacting the liquid medium for an effective period of time with a hydrogel composition comprising a hydrophilic polymer carrying multiple pendant sulphonyl groups, optionally with multiple pendant carboxylic groups, on each polymer molecule.

The expression "denutrifying" used herein refers in particular to removal of such nutrients that are essential for sustaining microbes from the liquid medium, to an extent which causes killing of microbes present in the medium. Preferably, the denutrifying is rapid in the sense that it takes place at a rate which causes rapid killing of the microbes in the liquid medium, as discussed above.

The nutrients include essential metals in soluble form, for example di- and tri-valent metal ions which may be in hydrated, solvated or chelated form. Such metal ions include, for example, Mg²⁺, Ca²⁺, Zn²⁺ and Fe³⁺. Chelated forms of the nutrients may, for example, include Fe³⁺-containing siderophores.

The liquid microbe-containing medium may, for example, be wound fluid, or wound bed fluid or wound biofilm.

Herein is also disclosed, a topical hydrogel composition comprising a hydrophilic polymer carrying multiple pendant sulphonyl groups, optionally with multiple pendant carboxylic groups, on each polymer molecule, for use in a method of rapidly killing microbes in an aqueous medium containing the same and/or rapidly denutrifying the aqueous medium, for example in the treatment of a chronic skin lesion, in a human or non-human mammal, particularly a human.

Herein is also disclosed, the use of a hydrogel composition comprising a hydrophilic polymer carrying multiple pendant sulphonyl groups, optionally with multiple pendant carboxylic groups, on each polymer molecule, in the preparation of a medicament for use in a method of rapidly killing microbes in an aqueous medium containing the same and/or rapidly denutrifying the aqueous medium, for example in a topical medicament for the treatment of a chronic skin lesion, in a human or non-human mammal, particularly a human.

The microbes generally include bacteria, fungi, yeasts or any mixture thereof. The method of the present disclosure will be particularly useful against pathogenic microbes that are present in clinics, hospitals and other medical treatment centres or care homes. The bacteria may be Gram-negative or Gram-positive bacteria.

Examples of bacteria that are killed by the method of the present disclosure include *Pseudomonas aeruginosa, Staphylococcus aureus* and *Escherichia coli.*

Examples of fungi that are killed by the method of the present disclosure include *Candida albicans* and *Aspergillus niger.*

*C. albicans* has yeast-like properties, and the efficacy of the method of the present disclosureagainst that organism is strongly indicative of more general activity against yeasts.

A wound to be treated using the present invention is a chronic ulcerous skin lesion, for example a malignant or pre-malignant chronic ulcerous skin lesion or a benign chronic ulcerous skin lesion.

The chronic ulcerous skin lesion may particularly be selected from venous leg ulcers, venous foot ulcers, arterial leg ulcers, arterial foot ulcers, decubitus ulcers (e.g. pressure sores, bedsores), diabetic ulcers, post-surgical ulcerous lesions and chronic burn lesions.

The chronic ulcerous skin lesion may be a high exudation lesion, a medium exudation lesion or a low exudation lesion.

The hydrogel composition has the capacity to absorb many times (e.g. at least about 2.5 times, for example at least about 5 times, for example at least about 10 times, for example between about 10 and about 50 times, and potentially up to about 250 times) its own weight of exudate or other fluid in 24 hours. Therefore, the exudate management capacity of the composition can be selected according to the intended target patients and lesions for treatment. The hydrogel preferably has a water activity greater than 0.4, for example greater than 0.5, for example greater than 0.6, for example greater than 0.7, preferably greater than 0.8, preferably greater than 0.9, preferably greater than 0.95, preferably greater than 0.97 but less than 0.99 in the absence of maceration. In the presence of maceration the hydrogel preferably has a water activity less than 0.95, more preferably less than 0.9. As mentioned below, in some instances the water activity of the hydrogel may be substantially lower than 0.4. As described in more detail below, one particularly suitable hydrogel for use in the present invention may have a water activity in the range of 0.6 to 0.89.

The present invention has been found to provide a wound healing and/or microbial kill effect in the absence of other antimicrobial agents (e.g. antibiotics) and/or painkilling agents.

Therefore, the aspects of the present invention as defined herein are suitably provided for use on subjects who, at the start of their treatment according to the present invention, are not receiving (and preferably also who have not been receiving recently, i.e. in the previous time period of about 2 weeks) other, separately administered, antimicrobial and/or painkilling agents, and more preferably still for use on subjects who, at the start of their treatment according to the present invention, are not receiving other antimicrobial and/or painkilling agents, whether separately administered or incorporated in the hydrogel.

Such other agents are typically so-called "small-molecule" (non-polymeric, non-protein) antimicrobial and/or painkilling agents (for example, having molecular weights less than about 1000). Such antimicrobial and/or painkilling agents may be available on a prescription- only basis, on a non-prescription-only basis, or on both of these bases. Such antimicrobial agents include antibiotics, such as for example antibiotics of the penicillin, cephalosporin, macrolide, aminoglycoside and teracycline families and combinations thereof. Such painkilling agents include analgesics of the narcotic and non-narcotic families and combinations thereof, such as for example nitrous oxide (Entonox), salicylates such as aspirin, acetaminophen, nonsteroidal anit-inflammatory drugs such as ibuprofen, opiates and opioids such as codeine, propoxyphene (e.g. Darvon and Wygesic), meperidine (Demerol) and morphine, acetaminophen/codeine (e.g. Tylenol with Codeine and Tylox), aspirin/codeine (e.g. Empirin with Codeine), propoxyphene/aspirin (e.g., Darvon Compund-65); and aspirin/caffeine/butalbital (Florinal).

Apart from immediately apparent cost advantages in avoiding the use of other antimicrobial and/or painkilling agents in the treatments, the present invention makes available new therapeutic applications by avoiding over-prescription of antibiotics (thereby reducing the risk of emergence of antibiotic-resistant strains or populations of bacteria), and opens effective wound treatments to subjects who are, or might be, sensitive, reactive or allergic to certain classes of antibiotics, painkillers or other bioactive agents, or who are addicted to or dependent on opiate or other painkillers (analgesics) conventionally used in conjunction with wound care (or who are actually or potentially susceptible to such addiction or dependence).

Furthermore, the application of the present invention to subjects who are, at the start of their treatment according to the present invention, not receiving (or have not been recently receiving) other, separately administered, antimicrobial and/or painkilling agents, is technically advantageous in that such patients have no psychological reliance on the antimicrobial and/or painkilling agents and therefore are psychologically receptive to the simpler treatment according to the present invention. The psychological receptiveness of a patient to the treatment about to be delivered can be an important factor in improving the clinical outcome for the patient, and can provide an unexpected and unquantifiable advantage in the treatment.

A similar psychological reliance can be observed in patients who are, at the start of treatment according to the present invention, receiving (or have recently been receiving) one or more other, different, hydrogel or hydrocolloid treatment for the same purpose (i.e. for the same wound). Therefore, the aspects of the present invention as defined herein are suitably provided for use on subjects who, at the start of their treatment according to the present invention, are not receiving (and preferably also who have not been receiving recently, i.e. in the previous time period of about 2 weeks) one or more other, different, hydrogel or hydrocolloid treatment for the same purpose.

As discussed in more detail below, we have shown that the beneficial effects of the hydrogel dressing of the present invention derive from the multiple pendant sulphonyl groups, optionally with multiple pendant carboxylic groups, of the polymer molecules acting *in situ* at the zone of contact with the wound to selectively concentrate one or more naturally exuded salts in the ulcerous region of the lesion (the "wound bed") and/or to selectively absorb one or more naturally exuded salts in the wound bed, without the need for externally applied salt or other ionic aqueous solutions, and preferably also in the absence of salt or other ionic aqueous solutions in the liquid held within the polymer matrix of the hydrogel, so that the blocking mechanism preventing completion of the normal wound healing process is overridden, bypassed, shut off or otherwise disabled, and continuation of the normal wound healing process to substantial completion is enabled or initiated.

The selectivity of the concentration of the naturally exuded salts is preferably achieved through the control of the counterion(s), present on the sulphonyl groups or present on the multiple sulphonyl and carboxylic groups. Generally speaking, it is believed that selection of, say, sodium counterions on -SO₃⁻ groups (i.e. a sulpho group in salt form) will favour concentration of sodium salts (e.g. sodium chloride) in the wound bed, whereas selection of, say, potassium counterions on -SO₃⁻ groups will favour concentration of potassium salts (e.g. potassium chloride) in the wound bed. For example, we believe that it will be advantageous for the molar ratio of sodium ions to potassium ions in the hydrogel composition to be in the range of between about 100:0 and about 100:10, for example between about 100:0.1 and about 100:5, for example between about 100:0.1 and about 100:1, for example between about 100:0.2 and about 100:0.8, or for example between about 100:1 and about 100:5.

From this, it is now possible to control the healing process in chronic ulcerous skin lesions, for the first time, without the need for externally applied salts or other bioactive agents apart from the dressing itself, and more particularly without the need for salts or other bioactive agents in the dressing apart from the hydrogel polymer matrix (including the associated water and the ions of the hydrogel polymer) of the dressing itself.

We believe that the present disclosure represents the first ever finding of a method of using and controlling the body's naturally exuded salt solutions, occurring in the wound bed, to assist the healing of chronic ulcerous skin lesions, without the need for externally applied salt solutions, and without the need for bioactive agents other than those which comprise the hydrogel polymeric matrix (including its associated water and ions) itself.

Therefore, herein is also disclosed, a method of concentrating one or more naturally exuded dissolved salts in a wound bed of a chronic ulcerous skin lesion, in a human or non-human mammal, particularly a human, to an extent sufficient to treat the skin lesion without the need for externally applied salt solutions, comprising contacting the skin lesion for an effective period of time with a topical hydrogel composition comprising a hydrophilic polymer carrying multiple pendant sulphonyl groups, optionally with multiple pendant carboxylic groups, on each polymer molecule.

Herein is also disclosed, a topical hydrogel composition comprising a hydrophilic polymer carrying multiple pendant sulphonyl groups, optionally with multiple pendant carboxylic groups, on each polymer molecule, for concentrating one or more naturally exuded dissolved salts in a wound bed of a in a human or non-human mammal, particularly a human, to an extent sufficient to treat the chronic skin lesion without the need for externally applied salt solutions.

Herein is also disclosed, the use of a hydrogel composition comprising a hydrophilic polymer carrying multiple pendant sulphonyl groups, optionally with multiple pendant carboxylic groups, on each polymer molecule in the preparation of a topical medicament, preferably a dressing, for a chronic ulcerous skin lesion, in a human or non-human mammal, the hydrogel composition containing sufficient pendant sulphonyl groups, optionally with multiple pendant carboxylic groups, to concentrate in use one or more naturally exuded salts in a wound bed, for example the wound bed of the chronic ulcerous skin lesion, to an extent sufficient to treat the chronic skin lesion without the need for externally applied salt solutions.

The concentration of one or more naturally exuded salt in a wound bed according to the present disclosure may be balanced by a dilution of one or more other naturally exuded salt in the wound bed, as a result of selective uptake and release of water and ions, respectively into and from the hydrogel composition.

The effective amount of pendant sulphonyl groups, optionally with multiple pendant carboxylic groups, in the hydrogel, for concentrating in use one or more naturally exuded salts in a wound bed to an extent sufficient to treat the chronic skin lesion without the need for externally applied salt solutions, will vary from subject to subject, but generally speaking the effective amount is as described in more detail below, in the section headed "Detailed Description of the Invention; The Hydrogel, Dressing and Treatment". Adjustments to the sulphonyl and optional carboxylic groups to suit individual subjects will be within the capacity of one skilled in the art, following simple experimental procedures.

Our work has also shown that the hydrogel composition used in the present invention has a remarkable and unexpected capacity to rapidly autolytically debride a skin lesion while the dressing is in place, thereby reducing the requirement for specific surgical, mechanical or chemical debridement procedures on the wound. To be able to reduce the requirement for these unpleasant, and often extremely painful, procedures is a major advance in the treatment of chronic wounds.

In the autolytic debridement achieved using the present invention, the necrotic tissue or dead debris of the skin lesion appears to be dissolved and removed (absorbed) into the hydrogel composition.

Therefore, herein is also disclosed, a method of rapidly autolytically debriding a dressed chronic ulcerous skin lesion, in a human or non-human mammal, particularly a human, comprising contacting the skin lesion for an effective period of time with a dressing comprising a topical hydrogel composition, the topical hydrogel composition comprising a hydrophilic polymer carrying multiple pendant sulphonyl groups, optionally with multiple pendant carboxylic groups, on each polymer molecule.

Herein is also disclosed, a topical hydrogel composition comprising a hydrophilic polymer carrying multiple pendant sulphonyl groups, optionally with multiple pendant carboxylic groups, on each polymer molecule, for rapidly autolytically debriding a a chronic ulcerous skin lesion, in a human or non-human mammal, particularly a human.

Herein is also disclosed, the use of a hydrogel composition comprising a hydrophilic polymer carrying multiple pendant sulphonyl groups, optionally with multiple pendant carboxylic groups, on each polymer molecule in the preparation of a topical medicament dressing for a chronic ulcerous skin lesion, in a human or non-human mammal, the hydrogel composition containing sufficient pendant sulphonyl groups, optionally with multiple pendant carboxylic groups, to rapidly autolytically debride a skin lesion dressed with the medicament dressing, for example a chronic ulcerous skin lesion, in a human or non-human mammal, particularly a human.

The expression "rapid autolytic debridement" used herein refers in particular to a speed of debridement which causes at least about an at least about 20 percent reduction, more particularly at least about 30 percent reduction, of the visible area of coverage by necrotic or dead tissue or slough within a period of about 20 days after application of the hydrogel composition.

The effective amount of pendant sulphonyl groups, optionally with multiple pendant carboxylic groups, in the hydrogel, for providing rapid autolytic debridement will vary from subject to subject, but generally speaking the effective amount is as described in more detail below, in the section headed "Detailed Description of the Invention; The Hydrogel, Dressing and Treatment". Adjustments to the sulphonyl and optional carboxylic groups to suit individual subjects will be within the capacity of one skilled in the art, following simple experimental procedures.

It is well known that water in hydrogels can be present in at least two forms, freezing and non-freezing, as measured by differential scanning calorimetry. In many examples of commercially available hydrogels the water is present only as non-freezing water. It has been found, however, that compositions with useful adhesive properties can be made which have both freezing and non-freezing water, and the water activity in such gels is generally high.

Our work has also shown that the hydrogel composition used in the present invention has a remarkable and unexpected capacity to normalise the condition of the skin formed over, and surrounding, a chronic ulcerous skin lesion, in a human or non-human mammal, particularly a human, more quickly and/or more completely in the lesion healing process than in a corresponding untreated skin lesion.

Therefore, herein is also disclosed a method of normalising the condition of the skin formed over, and surrounding, a chronic ulcerous skin lesion, in a human or non-human mammal, particularly a human, comprising contacting the skin lesion for an effective period of time with a topical hydrogel composition, the topical hydrogel composition comprising a hydrophilic polymer carrying multiple pendant sulphonyl groups, optionally with multiple pendant carboxylic groups, on each polymer molecule.

Herein is also disclosed, a topical hydrogel composition comprising a hydrophilic polymer carrying multiple pendant sulphonyl groups, optionally with multiple pendant carboxylic groups, on each polymer molecule, for normalising the condition of the skin formed over, and surrounding, a chronic ulcerous skin lesion, in a human or non-human mammal, particularly a human.

Herein is also disclosed, the use of a hydrogel composition comprising a hydrophilic polymer carrying multiple pendant sulphonyl groups, optionally with multiple pendant carboxylic groups, on each polymer molecule, in the preparation of a topical medicament, for example a dressing, for normalising the condition of the skin formed over, and surrounding a chronic ulcerous skin lesion, in a human or non-human mammal, particularly a human.

The term "normalising" used herein in relation to skin condition refers particularly to the reduction in hardness and scaliness of skin and its tendency to crack and bleed, to a skin texture characteristic of healthy, well-moistened, flexible and non-cracking skin. Scaly and cracking skin is often found in persons suffering from chronic skin wounds and poor circulation, and the elderly, and the term "normalising" in relation to skin condition will be well understood by those skilled in the art.

The effective amount of pendant sulphonyl groups, optionally with multiple pendant carboxylic groups, in the hydrogel, for normalising the condition of the skin formed over, and surrounding, a skin lesion will vary from subject to subject, but generally speaking the effective amount is as described in more detail below, in the section headed "Detailed Description of the Invention; The Hydrogel, Dressing and Treatment". Adjustments to the sulphonyl and optional carboxylic groups to suit individual subjects will be within the capacity of one skilled in the art, following simple experimental procedures.

Our work has also shown that the hydrogel composition used in the present invention has a remarkable and unexpected capacity to stimulate the healing process in the wound bed, including to promote granulation, of a skin lesion. Without being bound by theory, it is believed that the promotion and/or maintenance of granulation in the wound healing process is an important contributing effect underlying the present invention.

Herein is also disclosed, a method of stimulating the healing process in a wound bed of a chronic ulcerous skin lesion, in a human or non-human mammal, particularly a human, comprising contacting the skin lesion, for an effective period of time for promoting and/or maintaining granulation, with a topical hydrogel composition, the topical hydrogel composition comprising a hydrophilic polymer carrying multiple pendant sulphonyl groups, optionally with multiple pendant carboxylic groups, on each polymer molecule.

Herein is also disclosed, a topical hydrogel composition comprising a hydrophilic polymer carrying multiple pendant sulphonyl groups, optionally with multiple pendant carboxylic groups, on each polymer molecule, for promoting and/or maintaining granulation of a chronic ulcerous skin lesion, in a human or non-human mammal, particularly a human.

Herein is also disclosed, the use of a hydrogel composition comprising a hydrophilic polymer carrying multiple pendant sulphonyl groups, optionally with multiple pendant carboxylic groups, on each polymer molecule, in the preparation of a topical medicament, for example a dressing, for promoting and/or maintaining granulation of a chronic ulcerous skin lesion, in a human or non-human mammal, particularly a human.

The term "promoting and/or maintaining granulation of a skin lesion" used herein refers particularly to the initiation and health progression of the granulation phase of healing of a skin lesion.

The effective amount of pendant sulphonyl groups, optionally with multiple pendant carboxylic groups, in the hydrogel, for promoting and/or maintaining granulation of a chronic ulcerous skin lesion will vary from subject to subject, but generally speaking the effective amount is as described in more detail below, in the section headed "Detailed Description of the Invention; The Hydrogel, Dressing and Treatment". Adjustments to the sulphonyl and optional carboxylic groups to suit individual subjects will be within the capacity of one skilled in the art, following simple experimental procedures.

The hydrogel composition used in the present invention provides a moist environment to tissue (typically skin and wound tissue) in contact with it, so that the wound healing effects of the present invention are generally categorised as moist wound healing

The hydrogel composition suitably serves to selectively concentrate and absorb salts in the wound bed not only in the absence of externally applied salt solutions but also in the absence of salt solutions in the liquid held within the polymer matrix of the hydrogel. However, as explained below, other bioactive components may in some instances advantageously be present in the liquid held within the polymer matrix of the hydrogel.

The concentration and absorption of the naturally exuded salts in the wound bed is preferably achieved under external selective control through primarily or exclusively selection of the (dry) material of the dressing, preferably without the need for external media to be applied. In particular, selection of the counterion (cation) of -SO₃⁻ groups or -SO₃⁻ and COO⁻ groups of the hydrogel polymer enables the relative concentrations of particular salts to be controlled in the wound bed. Generally speaking, the distribution of salts in the wound be will relate to the distribution of the corresponding alkali metal ions as counterions for the pendant sulphonyl groups, across at least the surface polymer molecules of the hydrogel composition used in the present invention.

The ability of the hydrogel composition used in the present invention to concentrate naturally exuded salts in the wound bed is measurable by the change in osmolarity of an external medium induced by the hydrogel composition. It is most preferred that the hydrogel compositions used in the present invention induce an increase in the ionic osmolarity of an external salt solution by between about 2% and about 150% as measured in a standard osmometer (for example, a Roebling Automatik Osmometer) over a 24 hour period, and that this induced increase in osmolarity is accompanied over the same time period by an uptake of the external salt solution into the hydrogel at an amount of at least about 2.5 times the starting weight of the hydrogel composition. The hydrogel compositions may induce an increase in the non-ionic osmolarity of an external salt solution by between about 2% and about 1500% as measured in a standard osmometer (for example, a Roebling Automatik Osmometer) over a 24 hour period, and that this induced increase in osmolarity is accompanied over the same time period by an uptake of the external salt solution into the hydrogel at an amount of at least about 2.5 times the starting weight of the hydrogel composition.

The ability of the hydrogel composition used in the present invention to concentrate naturally exuded salts in the wound bed is measurable by the change in concentration of ions of an external medium induced by the hydrogel composition. It is most preferred that the hydrogel compositions used in the present invention induce an increase in the sodium ion concentration of an external salt solution comprising sodium and calcium ions by between about 0.1% and about 25% as measured by atomic absorption spectrometry (e.g. using a standard atomic absorption spectrometer; London and Scandinavian Metallurgical Co. Ltd., Rotherham, UK) over a 24 hour period, and that this induced increase in sodium ion concentration is accompanied over the same time period by an uptake of the external salt solution into the hydrogel at an amount of at least about 2.5 times the starting weight of the hydrogel composition

The ability of the hydrogel composition used in the present invention to absorb naturally exuded salts from the wound bed is measurable by the change in concentration of ions of an external medium induced by the hydrogel composition. It is most preferred that the hydrogel compositions used in the present invention induce a decrease in the multivalent ion concentration of an external salt solution comprising sodium, calcium and other multivalent ions (for example zinc) by between about 0.1% and about 90% as measured by atomic absorption spectrometry (e.g. using a standard atomic absorption spectrometer; London and Scandinavian Metallurgical Co. Ltd., Rotherham, UK) over a 24 hour period, and that this induced decrease in multivalent (e.g. calcium) ion concentration is accompanied over the same time period by an uptake of the external salt solution into the hydrogel at an amount of at least about 2.5 times the starting weight of the hydrogel composition.

In the absence of potassium counterions in the hydrogel it is most preferred that the hydrogel compositions used in the present invention induce a decrease in the potassium ion concentration of an external salt solution comprising sodium, calcium and potassium ions by no more than 80%, for example no more than about 60%, for example no more than about 30% as measured by atomic absorption spectrometry (e.g. using a standard atomic absorption spectrometer; London and Scandinavian Metallurgical Co. Ltd., Rotherham, UK) over a 24 hour period, and that this induced decrease in potassium ion concentration is accompanied over the same time period by an uptake of the external salt solution into the hydrogel at an amount of at least about 2.5 times the starting weight of the hydrogel composition.

In the presence of potassium counterions in the hydrogel it is most preferred that the hydrogel compositions used in the present invention induce a change in the potassium ion concentration of an external salt solution comprising sodium, potassium and calcium ions by between about - 90% and about +100%, for example between about -60% and about +10%, for example between about -60% and about 0%, for example between about -50% and about 0%, between about -40% and about 0%, or between about 0.1% and about 25% (- = decrease; + = increase), as measured by atomic absorption spectrometry (e.g. using a standard atomic absorption spectrometer; London and Scandinavian Metallurgical Co. Ltd., Rotherham, UK) over a 24 hour period, and that this induced change in sodium ion concentration is accompanied over the same time period by an uptake of the external salt solution into the hydrogel at an amount of at least about 2.5 times the starting weight of the hydrogel composition

The ability of the hydrogel composition used in the present invention to concentrate naturally exuded salts in the wound bed is preferably achieved rapidly on contact with the wound bed. It is most preferred that the hydrogel compositions used in the present invention induce an increase in the ionic osmolarity of an external salt solution by between about 2% and about 150% as measured in a standard osmometer (for example, a Roebling Automatik Osmometer) over a 24 hour period. Preferably at least 20% of the increase occurs within the first 2 hours, and preferably this induced increase in osmolarity is accompanied over the 2 hour time period by an uptake of the external salt solution into the hydrogel. For example, the external salt solution may be taken up over the first 2 hours at an amount of at least about 0.5 times the starting weight of the hydrogel composition. Preferably the induced increase in sodium ion concentration is accompanied over the 24 hour time period by an uptake and/or a further uptake of the external salt solution into the hydrogel. For example, the external salt solution may be taken up over the 24 hour period in an amount of at least about 2.5 times the starting weight of the hydrogel composition. The hydrogel compositions may induce an increase in the non-ionic osmolarity of an external salt solution by between about 2% and about 1500% as measured in a standard osmometer (for example, a Roebling Automatik Osmometer) over a 24 hour period, and that this induced increase in osmolarity is accompanied over the same time period by the same extent of uptake of the external salt solution into the hydrogel as mentioned above.

Wound dressings are typically kept in place for periods up to 7 days, and then changed. During this time the dressing may have absorbed substantial quantities of exudate and have undergone substantial changes in the balance of counterions within it, along with a dilution of the sulphonyl and/or any carboxylic groups present. In this state the hydrogel is partially of fully swollen. The ability of the hydrogel to concentrate ions in the wound bed and absorb ions from the wound bed will consequently change. Fluid exuded from the wound at times subsequent to the initial application of the hydrogel may have a salt composition similar to that at the beginning of the treatment and of a composition not conducive to wound healing. It is therefore important that the hydrogel continues to be able to absorb ions from the wound bed and concentrate ions in the wound bed at a level effective for wound healing.

Therefore, herein is disclosed, a method for changing the nature and/or concentration of dissolved ions in a liquid (e.g. and aqueous liquid such as wound exudate), comprising contacting the liquid with a hydrogel composition comprising a hydrophilic polymer carrying multiple pendant sulphonyl groups, optionally with multiple pendant carboxylic groups, on each polymer molecule, wherein the hydrogel composition has absorbed part or all of its full absorption capacity of fluid. The absorbed fluid is, for example, salt containing fluid or the external fluid.

The ability of the hydrogel composition used in the present invention that is partially or fully swollen by salt containing fluid to absorb naturally exuded salts in the wound bed is measurable by the change in concentration of ions of an external medium induced by the partially or fully swollen hydrogel composition. It is most preferred that the hydrogel compositions used in the present invention when used in a partially or fully swollen state induce an increase in the ionic osmolarity of an external salt solution by between about 2% and about 150% as measured in a standard osmometer (for example, a Roebling Automatik Osmometer) over a 24 hour period. The hydrogel compositions may induce an increase in the non-ionic osmolarity of an external salt solution by between about 2% and about 1500% as measured in a standard osmometer (for example, a Roebling Automatik Osmometer) over a 24 hour period.

The control of salt concentration in the wound bed is an important factor in pain relief, because of the relationship between the membrane potential at the pain neurons and the transmission of pain signals to the brain. Calcium uptake by nerve endings is also believed to be important in the transmission of pain signals. We believe that the hydrogel composition used in the present invention serves to control pain in a novel and hitherto unappreciated way.

The capacity of the present invention to reduce pain associated with a skin lesion and/or pain associated with removal of a wound dressing is surprising and remarkable, and leads to a reduction in the requirement for specific pain relief. Typically, hitherto very strong (e.g. opiate) pain relief can be required for chronic skin lesions, which can lead to opiate dependence and addiction, as well as the cost and inconvenience of administering the relief. To be able to reduce the requirement for such pain relief is a major advance in wound care, particularly the treatment of chronic wounds.

Therefore, herein is also disclosed, a method of reducing the pain of a chronic ulcerous skin lesion in a human or non-human mammal, particularly a human, comprising contacting the skin lesion for an effective period of time with a topical hydrogel composition comprising a hydrophilic polymer carrying multiple pendant sulphonyl groups, optionally with multiple pendant carboxylic groups, on each polymer molecule.

Herein is also disclosed, a topical hydrogel composition comprising a hydrophilic polymer carrying multiple pendant sulphonyl groups, optionally with multiple pendant carboxylic groups, on each polymer molecule, for reducing the pain of a chronic ulcerous skin lesion in a human or non-human mammal, particularly a human.

Herein is also disclosed, the use of a hydrogel composition comprising a hydrophilic polymer carrying multiple pendant sulphonyl groups, optionally with multiple pendant carboxylic groups, on each polymer molecule in the preparation of a topical medicament, preferably a dressing, for treating pain of a chronic ulcerous skin lesion in a human or non-human mammal.

Using the standard pain scale of 0 to 10 conventionally used in nursing in connection with chronic skin lesions, the present invention provides a substantial reduction of pain associated with the wound, the dressing, and the dressing-changing procedure, corresponding with the progress of healing of the wound. Reductions of 3 or more, for example, 4 or more, for example 5 or more, even between 6 and 9 points, on the scale have been observed. After substantially complete healing of the wound is achieved, the pain reported by the patient will reduce to zero. In one study of 15 subjects with chronic leg and foot ulcers receiving treatment according to the present invention for periods in the range of 4 to 6 weeks, significant reductions in average pain levels (p<0.01) from 8.25 at the start of the treatment to 3.25 after the treatment have been observed. In another study of 10 subjects with chronic leg and foot ulcers receiving treatment according to the present invention, reductions in average pain levels from 8.5 at the start of the treatment to 3.9 after the treatment have been observed.

The effective amount of pendant sulphonyl groups, optionally with multiple pendant carboxylic groups, in the hydrogel, for providing this level of pain relief will vary from subject to subject, but generally speaking the effective amount is as described in more detail below, in the section headed "Detailed Description of the Invention; The Hydrogel, Dressing and Treatment". Adjustments to the sulphonyl and optional carboxylic groups to suit individual subjects will be within the capacity of one skilled in the art, following simple experimental procedures.

The present disclosure thus provides an effective treatment of chronic skin lesions such as ulcerated skin lesions (e.g. chronic venous or arterial leg ulcers) or diabetic ulcers to promote their healing. Herein is also disclosed effective killing of microbes and broad relief of skin pain, as well as stimulation of the wound bed and wound granulation, autolytic debridement of wounds and skin conditioning.

The treatment makes available simultaneous reduction of one or more undesirable characteristics of a chronic skin lesion selected from pain associated with the wound, pain associated with changing of the dressing, exudation, malodour, irritation and hyperkeratosis.

Undesirable effects of conventional dressings for chronic skin lesions, for example maceration, incomplete absorption of exudate, excoriation, scarring of the final healed tissue, contact dermatitis, varicose eczema or skin stripping can be reduced using the present invention.

The dressing used in the present invention is easy to apply and change, with resultant cost savings and efficiency enhancements. Moreover, the number of dressing changes required is reduced substantially by the present invention. For example, a study of 20 chronic leg and foot ulcer cases treated using the present invention over periods of 4 to 6 weeks has shown that the average number of changes of dressings per week fell from 3.00 using conventional prior art dressings to 1.75 using the treatment according to the present invention.

Unless specifically stated otherwise, or implicitly otherwise by the context, the examples and preferences expressed herein in relation to any one aspect of the invention apply equally to all the other aspects of the invention, both independently of each other or in any combination.

To the extent that the prior use and printed publication of the ActiFormCool™ dressing mentioned above is found on examination to have made available to the public any aspect of the present invention or to have rendered any aspect of the present invention (or claim of the present application) obvious, thereby rendering patent protection unavailable under the relevant governing law, we hereby disclaim such use of the ActiFormCool™ dressing. In addition, we reserve the right to enter, for any territory or territories governed by that law, such a disclaimer explicitly into the claims of the present application and any subsequent application(s) and patent(s) derived therefrom.

### Detailed Description of the Invention

### The Hydrogel, Dressing and Treatment

The expression "hydrogel" and like expressions, used herein, are not to be considered as limited to gels which contain water, but extend generally to all hydrophilic gels, including those containing organic non-polymeric components in the absence of water. The gel forming agent may, for example, be selected from natural hydrophilic polymers, synthetic hydrophilic polymers, gelling hydrophilic biopolymers and all combinations thereof. The term "hydrogel" is used herein regardless of the state of hydration, and therefore includes, for example, hydrogels that are in a dehydrated or anhydrous state or in a state of partial hydration.

Hydrogels are described in greater detail in Hydrogels, Kirk-Othmer Encyclopedia of Chemical Technology, 4th Edition, vol. 7, pp. 783-807, John Wiley and Sons, New York.

The expression "polymer" and like expressions, used herein, includes homopolymers, copolymers and all mixtures and combinations thereof.

Hydrogels are, generally speaking, hydrophilic polymers characterized by their hydrophilicity (i.e capacity to absorb large amounts of fluid such as wound exudate) and insolubility in water: i.e. they are capable of swelling in water while generally preserving their shape.

The hydrophilicity is generally due to groups such as hydroxyl, carboxy, carboxamido, and esters, among others. On contact with water, the hydrogel assumes a swollen hydrated state that results from a balance between the dispersing forces acting on hydrated chains and cohesive forces that do not prevent the penetration of water into the polymer network. The cohesive forces are most often the result of crosslinking, but may result from electrostatic, hydrophobic or dipole-dipole interactions.

The hydrogels in the present invention include as a necessary component a hydrophilic polymer carrying multiple pendant sulphonyl groups on each polymer molecule.

Generally, the degree of sulphonylation of such a polymer is on average (number average) at least about one pendant sulphonyl group per linear 150 carbon atoms of the carbon atom backbone of the polymer, for example per linear 100 carbon atoms of the carbon atom backbone of the polymer, for example per linear 50 carbon atoms of the carbon atom backbone of the polymer, for example per linear 30 carbon atoms of the carbon atom backbone of the polymer, for example at least about one pendant sulphonyl group per linear 12 carbon atoms of the carbon atom backbone of the polymer, for example at least about one pendant sulphonyl group per linear six carbon atoms of the carbon atom backbone of the polymer. More preferably, the polymer will contain on average at least about two pendant sulphonyl groups per linear six carbon atoms of the carbon atom backbone of the polymer, for example up to about three pendant sulphonyl groups per linear six carbon atoms of the carbon atom backbone of the polymer. At the higher levels of sulphonylation it is preferred that pendant carboxylate groups will be substantially absent.

Most preferably, the polymer contains one pendant sulphonyl group per linear two carbon atoms of the carbon atom backbone of the polymer. Such a polymer is readily prepared by polymerising (meth)acrylic acid derivatives such as esters or amides using monomers containing one sulphonyl group per molecule. The sulphonyl groups are present in salt form, and may be covalently linked to the carbon atom backbone of the polymer. A suitable sulphonyl moiety is the -SO₃⁻ species, in salt form (-SO₃M), where M is a univalent metal counterion. Suitable linking moieties include alkylene bridges, alkylene-ester bridges, -O-bridges and alkylene-amide bridges. The alkylene moieties may be straight or branched, saturated and preferably contain from 1 to about 8 carbon atoms.

Such hydrophilic polymers include, for example, polymers derived from (meth)acryloyloxyalkylsulphonates salts, polymers of sulpho-substituted acrylamides such as acrylamidoalkanesulphonic acid salts, (for example, alkali salts), or any combination thereof. Mixtures of such polymers with each other are also envisaged.

Such polymers may, if desired, be used together with sulpho-free polymers. Such other polymers, if present, may suitably be selected from homopolymers or copolymers of acrylic and methacrylic acid esters, including hydroxyalkyl (meth)acrylates, 2-(N,N-dimethylamino)ethyl methacrylate, polymers and copolymers of other substituted and unsubstituted acrylamides, polymers and copolymers of N-vinylpyrrolidinone, and polyelectrolyte complexes.

The hydrophilic polymer carrying multiple pendant sulphonyl groups, optionally with multiple pendant carboxylic groups, on each polymer molecule should be present at least at the lesion-contacting surface of the hydrogel composition. If desired, the hydrophilic polymer carrying multiple pendant sulphonyl groups, optionally with multiple pendant carboxylic groups, on each polymer molecule may also be present in the internal bulk of the composition, and/or a sulphonyl-free polymer or combination of polymers may be present in the internal bulk of the composition.

Generally, the degree of carboxylation of such a polymer is on average (number average) at least about one pendant carboxylic group per linear 100 carbon atoms of the carbon atom backbone of the polymer, for example up to about one pendant carboxylic group per linear six carbon atoms of the carbon atom backbone of the polymer.

The hydrogel used in the present invention suitably comprises a substantially water-insoluble, slightly crosslinked, partially neutralized, gel-forming polymer material having the pendant sulphonyl groups, in salt form at least at its lesion-contacting surface. Such polymer materials can be prepared from polymerizable, unsaturated, acid- and ester-containing monomers. Any polymer to be present at the lesion-contacting surface of the composition will contain pendant sulphonyl groups, for example -SO₃⁻ in salt form, and optionally carboxylic groups in acid or salt form. Thus, such monomers include the olefinically unsaturated acids, esters and anhydrides which contain at least one carbon to carbon olefinic double bond. More specifically, these monomers can be selected from olefinically unsaturated carboxylic acids, carboxylic esters, carboxylic acid anhydrides; olefinically unsaturated sulphonic acids; and mixtures thereof.

Olefinically unsaturated carboxylic acid, carboxylic acid ester and carboxylic acid anhydride monomers include the acrylic acids typified by acrylic acid itself, methacrylic acid, ethacrylic acid, α-chloroacrylic acid, α-cyano-acrylic acid, β-methyl-acrylic acid (crotonic acid), α-phenyl acrylic acid, β-acryloxy-propionic acid, sorbic acid, α-chloro-sorbic acid, angelic acid, cinnamic acid, *p*-chloro-cinnamic acid, β-styryl-acrylic acid (1-carboxy-4-phenyl-1,3-butadiene), itaconic acid, citraconic acid, mesaconic acid, glutaconic acid, aconitic acid, maleic acid, fumaric acid, tricarboxy-ethylene and maleic acid anhydride and salts (e.g. alkali metal salts such as sodium, potassium and lithium salts) thereof. For forming any polymer to be present at the lesion-contacting surface of the composition, the monomer or monomer mixture will include a monomer containing pendant sulphonyl groups, e.g. -SO₃⁻ in salt form.

Olefinically unsaturated sulphonic acid monomers include aliphatic or aromatic vinyl sulphonic acids such as vinylsulphonic acid, allylsulphonic acid, vinyltoluenesulphonic acid and styrene sulphonic acid;; acrylic and methacrylic sulphonic acid such as sulphoethyl acrylate, sulphoethyl methacrylate, sulphopropyl acrylate, sulphopropyl methacrylate, 2-hydroxy-3-acryloxy propyl sulphonic acid, 2-hydroxy-3-methacryloxy propyl sulphonic acid and 2-acrylamido-2-methyl-propanesulphonic acid and salts (e.g. alkali metal salts, such as sodium, potassium) thereof. For forming any polymer to be present at the lesion-contacting surface of the composition, the monomer or monomer mixture will include a monomer containing pendant sulphonyl groups, e.g. -SO₃⁻ in salt form, wherein the salt form is sodium alone or a combination of sodium and potassium counterions, and optionally carboxylic groups in acid or salt form.

The monomers may suitably be used in admixture with each other or with other monomers. In one particularly useful embodiment of the invention, a monomer which has a first counter-cation associated with it may be used in admixture with one or more monomer which has/have one or more second/further counter-cation(s) associated with it/them. The monomers in their anionic form (i.e. disregarding the counter-cation) may be the same or different. In this way, the proportions of different cations (e.g. alkali metal ions such as sodium or potassium,) can be finely controlled in the resultant polymer (homopolymer or copolymer). The particular weight ratios of one monomer to the or each other monomer can be selected within wide limits by those skilled in the art, depending on the desired properties of the resultant hydrogel polymer.

Further examples of suitable monomers for use in the present invention include: a polyalkylene glycol acrylate or a substituted derivative thereof; a polyalkylene glycol methacrylate or a substituted derivative thereof; acrylic acid and salts thereof (e.g. alkali metal salts such as sodium, potassium and lithium salts); 2-acrylamido-2-methyl-propanesulphonic salts (e.g alkali metal salts, such as sodium, potassium); acrylic acid (3-sulphopropyl) ester salt (e.g. sodium or potassium salt); diacetone acrylamide (N-1,1-dimethyl-3-oxobutyl-acrylamide); a vinyl lactam (e.g. N-vinyl pyrrolidone or a substituted derivative thereof); an optionally substituted N-alkylated acrylamide such as hydroxyethyl acrylamide; and an optionally substituted N,N-dialkylated acrylamide; and/or N-acryloyl morpholine or a substituted derivative thereof. For forming any polymer to be present at the lesion-contacting surface of the composition, the monomer or monomer mixture will include a monomer containing pendant sulphonyl groups, e.g. -SO₃⁻ in salt form, and optionally carboxylic groups in acid or salt form.

The above monomers and monomer types may optionally include substituent groups. Optional substituents of the monomers used to prepare the hydrogels used in the present invention may preferably to selected from substituents which are known in the art or are reasonably expected to provide polymerisable monomers which form hydrogel polymers having the properties necessary for the present invention. Suitable substituents include, for example, lower alkyl, hydroxy, halo and amino groups.

In one particular form of the present invention, the hydrogel material may be free of uncrosslinked polymerised styrene sulphonates. In another particular form of the present invention, the hydrogel material may be free of any styrene sulphonate component, whether polymerised or unpolymerised and whether crosslinked or uncrosslinked.

The hydrogel used in the present invention preferably comprises a flexible three-dimensional polymer matrix. The hydrogel may be present in a composite in association with one or more other component selected from other hydrogels, hydrocolloids and non-hydrogel polymers, for example a polyurethane hydrogel. The hydrogel or composite may be a plasticised three-dimensional matrix of cross-linked and/or entangled polymer molecules, and preferably has sufficient structural integrity to be self-supporting even at very high levels of internal water content, with sufficient flexibility to conform to the surface contours of mammalian, preferably human, skin or other surface with which it is in contact.

The hydrogel generally comprises, in addition to the cross-linked polymeric network, an aqueous or non-aqueous plasticising medium including an organic plasticiser. This plasticising medium is preferably present in the same precursor solution as the monomer(s).

The hydrogel or composite (e.g. a composite with a polyurethane hydrogel) or any portion thereof may be present as a foam, i.e. including a rigid cellular internal structure. Methods for obtaining such hydrogels are disclosed, for example, in WO-A-03/077964.

The hydrogel composition may suitably be present as a thin sheet, preferably supported by a sheet support member to provide mechanical strength. The sheet support member for the hydrogel may, for example, be a thin scrim or net structure, for example formed of a synthetic and/or natural polymer such as polyethylene or polypropylene. The sheet support member for the hydrogel may overlie the hydrogel sheet on the major face of the sheet directed away from the lesion in use, or may be embedded within the hydrogel polymer. The sheet support member may, if desired, extend beyond the margins of the hydrogel composition, and may be provided with a skin adhesive portion to secure the dressing to the skin. The skin adhesive portion may be hydrogel in nature (for example a plasticised tacky hydrogel, which may be the same as or different from the hydrogel provided on the support member for the treatment according to the present invention), or may be another type of skin adhesive selected from the many skin adhesives known in the wound dressings art.

The hydrogel sheet may be part of a multi-layer composite, including further layers such as further hydrogels and/or other polymers and/or other sheet support members. For example, a breathable (air and/or moisture permeable) polymeric film (e.g. of polyurethane), which may if desired be present as a foam, may overlie the hydrogel sheet or composite on the major face of the sheet or composite directed away from the lesion in use.

The hydrogel composition and other sheet components as desired may preferably be provided with a release layer (e.g. of non-stick paper or plastic, such as siliconised paper or plastic) to protect one or both major face of the sheet prior to use.

The hydrogel composition and other sheet components as desired can constitute a dressing for the chronic ulcerous skin lesion which can, after removal of any release layer as appropriate, be applied to the lesion directly so that the major face which presents at its surface the hydrogel carrying pendant sulphonyl groups is directed towards the lesion and contacts the lesion, preferably the wound bed and surrounding tissues.

If desired, conventional bandages, cloths or other protective fabrics or materials can subsequently be applied to encase the dressing and hold it in place on the lesion.

Particularly where the hydrogel is plasticised, there is very slight adhesion between the hydrogel dressing and the patient's skin or the lesion tissue. This has the beneficial effect that one nurse or other healthcare professional can apply the dressing and can then prepare any desired bandages, cloths or the like for subsequent application. The dressing of the present invention will remain in place because of the mild adhesion, even if the patient moves before the further bandages etc. are applied.

The precursor liquid can comprise a solution of the gel-forming polymer in a relatively volatile solvent, whereby the hydrogel is deposited as a residue on evaporation of the solvent, or - more preferably - the precursor liquid will comprise a solution of the monomer(s), cross-linking agent, plasticiser, and optionally water and other ingredients as desired, whereby the hydrogel is formed by a curing reaction performed on the precursor liquid after application to the substrate to which the hydrogel is to be applied.

### Preparation of the Hydrogel and Dressing

In the following discussion, the second form of precursor solution and application protocol (*in situ* polymerisation of the hydrogel) will be discussed. The solvent deposition method carried out on a pre-formed gel-forming polymer is well known and the details of that procedure do not need to be reproduced here.

The polymerisation reaction is preferably a free-radical polymerisation with cross-linking, which may for example be induced by light, heat, radiation (e.g. ionising radiation), or redox catalysts, as is well known.

For example, the free radical polymerisation may be initiated in known manner by light (photoinitiation), particularly ultraviolet light (UV photoinitiation); heat (thermal initiation); electron beam (e-beam initiation); ionising radiation, particularly gamma radiation (gamma initiation); non-ionising radiation, particularly microwave radiation (microwave initiation); or any combination thereof. The precursor solution may include appropriate substances (initiators), at appropriate levels, e.g. up to about 5% by weight, more particularly between about 0.002% and about 2% by weight, which serve to assist the polymerisation and its initiation, in generally known manner.

Preferred photoinitiators include any of the following either alone or in combination:
Type I-α-hydroxy-ketones and benzilidimethyl-ketals e.g. Irgacure 651 (2,2-dimethoxy-2-phenylacetophenone). These are believed on irradiation to form benzoyl radicals that initiate polymerisation. Photoinitiators of this type that are preferred are those that do not carry substituents in the para position of the aromatic ring.

Preferred photoinitiators are 1-hydroxycyclohexyl phenyl ketone, for example as marketed under the trade name Irgacure 184 by Ciba Speciality Chemicals; Irgacure 651 (2,2-dimethoxy-2-phenylacetophenone); Darocur 1173 (2-hydroxy-2-propyl phenyl ketone); and mixtures of Irgacure 184 and Darocur 1173.

Photo-polymerisation is particularly suitable, and may be achieved using light, optionally together with other initiators, such as heat and/or ionising radiation. Photoinitiation will usually be applied by subjecting the pre-gel reaction mixture containing an appropriate photoinitiation agent to ultraviolet (UV) light. The incident UV intensity, at a wavelength in the range from 240 to 420nm, is typically greater than about 10mW/cm². The processing will generally be carried out in a controlled manner involving a precise predetermined sequence of mixing and thermal treatment or history.

The UV irradiation time scale should ideally be less than 60 seconds, and preferably less than 10 seconds to form a gel with better than 95% conversion of the monomers. Those skilled in the art will appreciate that the extent of irradiation will be dependent on a number of factors, including the UV intensity, the type of UV source used, the photoinitiator quantum yield, the amount of monomer(s) present, the nature of the monomer(s) present and the presence of polymerisation inhibitor.

The precursor solution (pre-gel) containing the monomer(s) and preferably cross-linking agent, water, plasticiser, photoinitiator and optionally other components as described below, is initially laid down on a substrate. Where the hydrogel composition is to be prepared in sheet for, the substrate will be a sheet. It may suitably comprise a release layer and any desired sheet support member that may be interposed between the release layer and the hydrogel composition, or embedded withing the hydrogel composition, in the finished dressing. In this way, the precursor solution can be polymerised *is situ* on the release layer, preferably with all or substantially all other components of the final dressing in place.

In one preferred embodiment, (on the one hand) the precursor solution in contact with the substrate to which it is to be applied and (on the other hand) the source of the polymerisation initiator (e.g. the radiation source) may move relative to one another for the polymerisation step. In this way, a relatively large amount of polymerisable material can be polymerised in one procedure, more than could be handled in a static system. This moving, or continuous, production system is preferred.

After completion of the polymerisation, the product is preferably sterilised in conventional manner. The sterile composite may be used immediately, e.g. to provide a skin-adhesive layer in an article, or a top release layer may be applied to the composite for storage and transportation of the composite.

If desired, certain ingredients of the hydrogel may be added after the polymerisation and optional cross-linking reaction. However, it is generally preferred that substantially all of the final ingredients of the hydrogel are present in the precursor solution, and that - apart from minor conventional conditioning or, in some cases, subsequent modifications caused by the sterilisation procedure - substantially no chemical modification of the hydrogel takes place after completion of the polymerisation reaction.

### Monomers

The monomers are discussed in more detail above. Particularly preferred monomers include: the sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A); the potassium salt of 2-acrylamido-2-methylpropane sulphonic acid (Potassium AMPS), which is available commercially at present from Lubrizol; acrylic acid (3-sulphopropyl) ester potassium salt, commonly known as SPA or SPAK (SPA or SPAK is available commercially in the form of a pure solid from Raschig); acrylic acid (3-sulphopropyl) ester sodium salt, commonly known as SPANa (SPANa is available commercially in the form of a pure solid from Raschig); and SPDA. Acrylic acid (BASF) may be used as supplied or in partial or complete salt form where the salt counterion is an alkali metal (e.g. sodium or potassium), alkaline earth metal (e.g. calcium) or ammonium. Mixtures of any two or more of the above monomers may be used. When a mixture of the monomers is used, it may, for example, be a mixture of NaAMPS and SPAK, a mixture of NaAMPS and SPANa, a mixture of NaAMPS and Potassium AMPS, a mixture of NaAMPS and Ammonium AMPS, or a mixture of NaAMPS and acrylic acid. The relative amounts of the monomers in a mixture will be dictated by the desired ratio of counterions (e.g. potassium, sodium and ammonium) in the hydrogel, as well as the required properties of the copolymer, and may be selected easily by those skilled in the art, if necessary with routine testing of the copolymers prepared. See the discussion above (page 16, first full paragraph), for information as to suitable molar ratios of sodium to potassium ions.

### Cross-linking Agents

Conventional cross-linking agents are suitably used to provide the necessary mechanical stability and to control the adhesive properties of the hydrogel. The amount of cross-linking agent required will be readily apparent to those skilled in the art such as from about 0.01% to about 0.5%, particularly from about 0.05% to about 0.4%, most particularly from about 0.08% to about 0.3%, by weight of the total polymerisation reaction mixture. Typical cross-linkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide.

### Organic Plasticisers

The one or more organic plasticiser, when present, may suitably comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is the preferred plasticiser. An alternative preferred plasticiser is the ester derived from boric acid and glycerol. When present, the organic plasticiser may comprise up to about 45%, for example up to about 35%, for example up to about 25%, for example up to about 15%, by weight of the hydrogel composition.

### Surfactants

Any compatible surfactant may optionally be used as an additional ingredient of the hydrogel composition. Surfactants can lower the surface tension of the mixture before polymerisation and thus aid processing. The surfactant or surfactants may be non-ionic, anionic, zwitterionic or cationic, alone or in any mixture or combination. The surfactant may itself be reactive, i.e. capable of participating in the hydrogel-forming reaction. The total amount of surfactant, if present, is suitably up to about 10% by weight of the hydrogel composition, preferably from about 0.05% to about 4% by weight.

The surfactant may, for example, comprise at least one propylene oxide/ethylene oxide block copolymer, for example such as that supplied by BASF Plc under the trade name Pluronic P65 or L64.

### Other additives

The hydrogel in the composite of the present invention may include one or more additional ingredients, which may be added to the pre-polymerisation mixture or the polymerised product, at the choice of the skilled worker. Such additional ingredients are selected from additives known in the art, including, for example, water, organic plasticisers, surfactants, polymeric material (hydrophobic or hydrophilic in nature, including proteins, enzymes, naturally occurring polymers and gums), synthetic polymers with and without pendant carboxylic acids, electrolytes, osmolites, pH regulators, colorants, chloride sources, bioactive compounds and mixtures thereof. The polymers can be natural polymers (e.g. xanthan gum), synthetic polymers (e.g. polyoxypropylene-polyoxyethylene block copolymer or poly-(methyl vinyl ether alt maleic anhydride)), or any combination thereof. By "bioactive compounds" we mean any compound or mixture included within the hydrogel for some effect it has on living systems, whether the living system be bacteria or other microorganisms or higher animals such as the patient. Bioactive compounds that may be mentioned include, for example, pharmaceutically active compounds, antimicrobial agents, antiseptic agents, antibiotics and any combination thereof. Antimicrobial agents may, for example, include: souces of oxygen and/or iodine (e.g. hydrogen peroxide or a source thereof and/or an iodide salt such as potassium iodide) (see, for example Bioxzyme™ technology, for example in The Sunday Telegraph (UK) 26 January 2003 or the discussion of the Oxyzyme™ system at www.wounds-uk.com/posterabstracts2003.pdf); honey (e.g. active Manuka honey); antimicrobial metals, metal ions and salts, such as, for example, silver-containing antimicrobial agents (e.g. colloidal silver, silver oxide, silver nitrate, silver thiosulphate, silver sulphadiazine, or any combination thereof), hyperchlorous acid; or any combination thereof.

In the Bioxzyme system, a dressing comprises two hydrogels. One contains glucose based antibacterial compounds and the other contains enzymes that convert the glucose into hydrogen peroxide. When these are exposed to air and contacted together at a wound site, the enzyme-containing gel being adjacent the skin and the glucose-containing gel overlying the enzyme-containing gel, a low level steady flow of hydrogen peroxide is produced, which inhibits anaerobic bacteria. This antibacterial effect can be enhanced by the inclusion of a very low level of iodide (less than about 0.04%) in the hydrogel. The hydrogen peroxide and the iodide react to produce iodine, a potent antimicrobial agent.

Hydrogels incorporating antimicrobial agents may, for example, be active against such organisms as *Staphylococcus aureus* and *Pseudomonas aeruginosa.*

Agents for stimulating the healing of wounds and/or for restricting or preventing scarring may be incorporated into the hydrogel. Examples of such agents include growth factors such as TGF (transforming growth factor), PDGF (platelet derived growth factor), KGF (keratinocyte growth factor, e.g. KGF-1 or KGF-2), VEGF (vascular endothelial growth factor), IGF (insulin growth factor, optionally in assiciation with one or more of IGF binding protein and vitronectin), e.g. from GroPep Ltd, Australia or Procyte, USA (see, e.g. WO-A-96/02270); cell nutrients (see, e.g., WO-A-93/04691); glucose (see, e.g., WO-A-93/10795); an anabolic hormone or hormone mixture such as insulin, triiodothyronine, thyroxine or any combination thereof (see, e.g., WO-A-93/04691); or any combination thereof.

Additional polymer(s), typically rheology modifying polymer(s), may be incorporated into the polymerisation reaction mixture at levels typically up to about 10% by weight of total polymerisation reaction mixture, e.g. from about 0.2% to about 10% by weight. Such polymer(s) may include polyacrylamide, poly-NaAMPS, polyethylene glycol (PEG), polyvinylpyrrolidone (PVP) or carboxymethyl cellulose.

Additional osmolite(s) may be included to modify the osmolarity of the hydrogel. Osmolites may be ionic (e.g. electrolytes, for example salts which are readily soluble in the aqueous phase of the hydrogel to increase the ionic strength of selected cations or anions and hence the osmolarity of the hydrogel). By selecting the ions present in an ionic osmolite, and particularly by selecting the cation so as to correspond or not with cationic counterions in the monomer(s) of the hydrogel, the ionic strength of certain anions (e.g. chloride) can be varied with fine control, without substantially changing the ionic strength of cations already present in very large amounts as counterions of the monomer(s).

Osmolites may be organic (non-ionic), for example organic molecules which dissolve in or intimately mix with the aqueous phase of the hydrogel to increase the osmolarity of the hydrogel deriving from non-ionic species in the aqueous phase. Such organic osmolites include, for example, water-soluble sugars (e.g. glucose, fructose and other monosaccharides; sucrose, lactose, maltose and other disaccharides; or any combination of mono- and di-saccharides), polyhydric alcohols (e.g. glycerol and other polyhydroxylated alkanols).

Additive ingredients may serve more than one purpose. For example, glycerol may serve as an organic plasticiser and an osmolite.

The hydrogel used in the present invention preferably consists essentially of a cross-linked hydrophilic polymer of a hydrophilic monomer and optionally one or more comonomer, together with water and/or one or more organic plasticiser, and optionally together with one or more additives selected from surfactants, polymers, pH regulators, electrolytes, osmolites, chloride sources, bioactive compounds and mixtures thereof, with less than about 40%, for example less than about 10%, by weight of other additives.

For further details of suitable hydrogel material for use in the present invention, and its preparation, please refer to the following publications: PCT Patent Applications Nos. WO-97/24149, WO-97/34947, WO-00/06214, WO-00/06215, WO-00/07638, WO-00/46319, WO-00/65143 and WO-01/96422.

The water activity of the hydrogel or of the precursor solution (as measured, for example, by a chilled mirror dewpoint meter, Aqualab T3) is preferably between 0.05 and 0.99, more preferably between, 0.2 and 0.99, and even more preferably between 0.3 and 0.98, for example between 0.6 and 0.89. The osmolarity of the precursor solution can therefore be used to optimise the hydrogel properties.

### Selective Control of Salts in the Wound Bed

The data and discussions included herein show that the present invention may enable selective control of the accumulation and concentration of naturally exuded salts in the wound bed.

The evidence points towards a salt effect, which is related to the sulphonyl groups of the hydrogel polymer.

Without wishing to be bound by theory, rejection of ions probably occurs as a result of a Donnan exclusion mechanism. This arises from the presence of a high concentration of fixed anionic charges associated with the sulphonyl groups in the polymer. As a result, there is an electrostatic repulsion to mobile anions trying to enter the gel from the swelling medium. Because overall electroneutrality is required, cations (for example sodium) will also be repulsed, giving rise to a slight increase in the osmolarity of the external swelling medium. The sulphonate group is seen to be an important controlling species because, in addition to having a large hydrodynamic volume which enhances water solubility, it will also be fully ionised at all physiological pHs due to its very low pKa, typically less than 2.

Furthermore, many hydrogels contain carboxylate groups, but those that contain only carboxylates (e.g. the alginate gels) do not show the advantages found in the present invention. From this it follows that carboxylate alone may not provide a controlling function.

Therefore, the evidence points strongly to the sulphonyl groups of the polymers used in the present invention being the controlling species. We consider, however, that the balance of carboxylation and sulphonation in the pendant groups of the hydrophilic polymers is likely to be important, as stated above.

### Brief Description of the Drawings

In the accompanying drawings:
Figure 1 shows the results of the experiment described in Example 19;
Figures 2 to 4 show the debridement effects of the treatment described in Example 21,
Figures 5 to 7 illustrate slough management effects of the treatment described in Example 21;
Figures 8 and 9 illustrate wound bed stimulation and pain reduction effects of the treatment described in Example 22;
Figure 10 shows the hydration effects of various hydrogels on the skin, as described in Example 23;
Figure 11 shows the improvement in skin condition achieved by the treatment of Example 23;
Figure 12 shows schematically the apparatus used in the experiment described in Example 27; and
Figure 13 shows the results of the experiment described in Example 27.

### Examples and Detailed Description of the Drawings

The following non-limiting examples are provided as further illustration of the present invention, but without limitation.

In the following Examples, and throughout this description, parts and percentages are by weight unless otherwise stated.

### Examples 1, 2, 4, 5, 6, 8, 9 and 10 and Reference Examples 3, 7, 11 to 15 - Hydrogel Compositions

Examples 1, 2, 4, 5, 6, 8, 9 and 10 illustrate suitable hydrogel composition which may be used with suitable sheet support members as described herein to provide a dressing for use in the present invention.

In these examples, each of the pre-gel formulations was cured as 0.3 to 2.6 kg per square metre coat weight by a medium pressure mercury arc lamp (GEW, UK).

### Example 1

Pre-gel: 70 parts by weight of 58% aqueous solution of the sodium salt of acrylamidomethylpropanesulphonic acid (Na AMPS, LZ2405 Lubrizol), 30 parts glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals)

### Example 2

Pre-gel: 52 parts by weight of 58% aqueous solution of the sodium salt of acrylamidomethyl-propanesulphonic acid (NaAMPS, LZ2405 Lubrizol), 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Reference Example 3

Pre-gel: 30 parts by weight acrylic acid (3-sulphopropyl) ester potassium salt, commonly known as SPA or SPAK (SPA or SPAK is available commercially in the form of a pure solid from Raschig), 70 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 4

Pre-gel: 52 parts by weight of 58% aqueous solution of the sodium salt of acrylamidomethyl-propanesulphonic acid (NaAMPS, LZ2405 Lubrizol), 3 parts acrylic acid (3-sulphopropyl) ester potassium salt, commonly known as SPA or SPAK (SPA or SPAK is available commercially in the form of a pure solid from Raschig 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 5

Pre-gel: 52 parts by weight of 58% aqueous solution of the sodium salt of acrylamidomethyl-propanesulphonic acid (NaAMPS, LZ2405 Lubrizol), 3 parts acrylic acid (3-sulphopropyl) ester potassium salt, commonly known as SPA or SPAK (SPA or SPAK is available commercially in the form of a pure solid from Raschig 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 6

Pre-gel: 52 parts by weight of 58% aqueous solution of the sodium salt of acrylamidomethyl-propanesulphonic acid (NaAMPS, LZ2405 Lubrizol), 1 parts acrylic acid (3-sulphopropyl) ester potassium salt, commonly known as SPA or SPAK (SPA or SPAK is available commercially in the form of a pure solid from Raschig 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Reference Example 7

Pre-gel: 26 parts by weight of 58% aqueous solution of the sodium salt of acrylamidomethyl-propanesulphonic acid (NaAMPS, LZ2405 Lubrizol), 15 parts acrylic acid (3-sulphopropyl) ester potassium salt, commonly known as SPA or SPAK (SPA or SPAK is available commercially in the form of a pure solid from Raschig 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 8

Pre-gel: 52 parts by weight of 58% aqueous solution of the sodium salt of acrylamidomethyl-propanesulphonic acid (NaAMPS, LZ2405 Lubrizol), 38 parts water, 10 parts Glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Examples 9 and 10 and Reference Examples 11 to 15

Pre-gel: 52 parts by weight of a mixture of X parts by weight of 58% aqueous solution of the sodium salt of acrylamidomethyl-propanesulphonic acid (NaAMPS, LZ2405 Lubrizol) and y parts by weight of 58% aqueous solution of acrylic acid (3-sulphopropyl) ester potassium salt, commonly known as SPA or SPAK (SPA or SPAK is available commercially in the form of a pure solid from Raschig 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals). X and y vary according to table below.

| | NaAMPS | SPA |
|---|---|---|
| Example | X | y |
| 9 | 100 | 0 |
| 10 | 97 | 3 |
| 11* | 94 | 6 |
| 12* | 85 | 15 |
| 13* | 70 | 30 |
| 14* | 50 | 50 |
| 15* | 39 | 61 |

| | | |
|---|---|---|
| *= Reference Example | | |

### Example 16 - Osmolarity Testing

The osmolarities of saline solutions before and after immersion for 24 hours were determined by freezing point depression (Roebling Automatik Osmometer; Camlab, Cambridge, UK). The saline solution A was made by weighing out 8.3 grams of sodium chloride and 0.28 grams of calcium chloride into a 21 glass (pyrex) beaker and making up to 11 with deionised water.

Samples of the hydrogels prepared according to Examples 1, 2, 4, 5 and 6and Reference Examples 3 and 7 (designated materials (a)), as well as reference samples of (b) polyethyleneglycol (PEG) gel, (c) a hydrocolloid gel, Granuflex™, (d) a cellulose gel (X-Cell™), and (e) a calgium alginate gel, in each case approximately 4g in weight, were immersed in 100ml of saline for 24 hours.

The osmolarities of the saline solutions before and after the immersion of gels for 24 hours were determined by freezing point depression (Roebling Automatik Osmometer; Camlab, Cambridge, UK). Typically 100 microlitres of solution was dispensed into an epindorf cartridge using a Gilsen pipette. The cartidge was then placed on the osmometer and a reading taken. The starting solution typically gave a reading about 271 mosm and HPLC grade de-ionised water 0.

All of the materials (a) (Examples 1, 2, 4, 5 and 6 in accordance with the present invention), including the hydrogels prepared according to both Examples 1 and 2, gave an increase in the saline osmolarity in the range 2-150 %. In the case of the glycerol-free hydrogels (a) the increase was less than 15%.

The reference materials (b), (d) and (e) gave a decrease in the saline osmolarity. Reference material (c) gave a small increase (+3%).

### Example 17 - Saline Uptake Testing

Materials (a) to (d) from Example 16 were tested also for the extent of their uptake of the saline medium (Saline Solution A).

The materials (a) gave a saline uptake of over 1000% by weight (i.e. the weight of saline taken up was over 10 times the weight of the initial hydrogel).

Reference materials (b) to (d) showed a saline uptake of less than about 270% in all cases (i.e. the weight of saline taken up was less than 3 times the weight of the initial gel)

From Examples 16 and 17 we see that the sulpho-containing hydrogels used in the present invention combine very high levels of uptake of external fluid with an increase in the osmolarity of the external saline.

In a glycerol-free system, the only way that this effect can be achieved is by an ion-exclusion mechanism in the process whereby the hydrogel materials imbibe external fluids. In a glycerol system, the mechanism may be different, but wherever glycerol is present in the hydrogels used in the present invention the sulphonyl moieties are also present so the ion-exclusion effect must be controlling.

There is evidence that such an ion-exclusion effect will be ion-specific. Selection of a particular counterion in a salt form of the pendant sulphonyl groups of the hydrogel will favour exclusion of that counterion from the imbibing process.

In the context of the wound bed of a chronic ulcerous skin lesion, and taking the Donnan effect into account (which shows ionic pairing of cations and anions must be maintained across a membrane), this result shows that the sulphonyl-containing hydrogels used in the present invention must tend to increase the concentration of dissolved inorganic salts in the portion of exudate that remains in the wound bed and not imbibed into the hydrogel.

### Example 18 - Atomic Absorption Spectrometry (Na and Ca)

In this example, atomic absorption spectrometry was used to determine sodium and calcium content of solutions. The starting saline solution used was Solution A.

The sodium and calcium content of the saline solutions before and after the immersion of the various gels and similar materials listed in the following table for 24 hours were determined by Atomic Absorption Spectrometry as undertaken by London and Scandinavian Metallurgical Co. Limited, Rotheram, UK.

### Electrolyte Compositions of supernatant solutions

| | After 24 hours Immersion | |
|---|---|---|
| Sample | Na ppm | Ca ppm |
| Ca Saline (Starting Solution) | 3329 | 103 |
| Example 2 gel | 3521 | 31 |
| Example 8 gel | 3645 | 38 |
| Example 1 gel | 3809 | 44 |
| Granuflex (hydrocolloid) | 3260 | 83 |
| Calcium Alginate (Sorbsan) | 3104 | 946 |
| Polyethylene Glycol | 3135 | 99 |
| Cellulose Dressing (X-Cell) | 3186 | 100 |

### Example 19 - Na, K and Ca

In this example, atomic absorption spectrometry was used to determine sodium, potassium and calcium content of solutions. The saline solution (solution B) used in this study was made by weighing out 5.6 grams sodium chloride, 0.013 grams potassium chloride and 0.24 grams calcium chloride (anhydrous) (all from Aldrich) into a 21 glass (pyrex) beaker and making up to 11 with deionised water.

The sodium, potassium and calcium content of the saline solutions before and after the immersion of the various gels and similar materials listed in the following table for 24 hours were determined by atomic absorption spectrometry as undertaken by London and Scandinavian Metallurgical Co Limited, Rotheram, UK.

| Sample | Na ppm | K ppm | Ca ppm |
|---|---|---|---|
| Starting Saline(B) | 2662 | 66 | 88 |
| Example 9 gel | 3160 | 43 | 23 |
| Example 10 gel | 3088 | 95 | 24 |
| Example 11 gel* | 3032 | 154 | 25 |
| Example 12 gel* | 2878 | 300 | 27 |
| Example 13 gel* | 2719 | 577 | 32 |
| Example 14 gel* | 2565 | 924 | 35 |
| Example 15 gel* | 2533 | 1083 | 33 |

| | | | |
|---|---|---|---|
| *= Reference Example | | | |

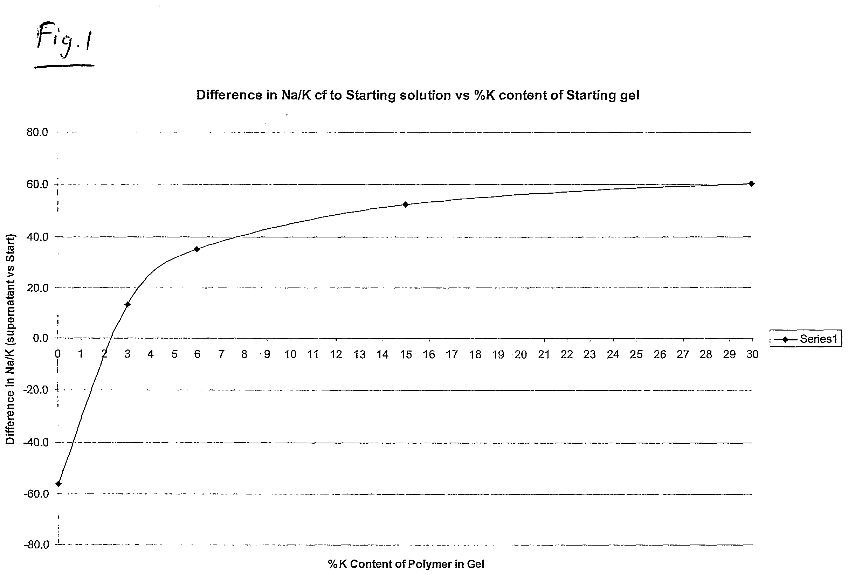

The results for the gels of Examples 9 to 10 and Reference Examples 11 to 13 were also used to prepare in Figure 1 of the accompanying drawings. Example 9 is shown as "0 % K Content" on the horizontal axis, Example 10 as "3 %", Reference Example 11 as "6 %", Reference Example 12 as "15 %", and Reference Example 13 as "30 %". In the vertical axis represents the change in the supernatant sodium/potassium ratio (ppm) observed between the start and the end of the experiment for the five hydrogels.

The data shows that the ion-exclusion effect is ion-specific. Surprisingly, the effect of the hydrogel in relation to exclusion of sodium ions is non-linear with respect to the concentration of potassium ions in the gel. Selection of a particular counterion in a salt form of the pendant sulphonyl groups of the hydrogel will favour exclusion of that counterion from the imbibing process as shown by the increasing rejection of potassium as the sulphopropyl acrylate content of the gel is increased.

### Example 20 - Summary of the "Cool₂O Plus" Clinical Study

The purpose of this study was to evaluate the potential and efficacy of the hydrogel of Example 2 plus dressing (referred to as "Cool₂O Plus") in achieving wound debridement and potential for closure in three common types of wounds found in patients in the community.

### Methods

Wound healing prognosis is difficult to predict. However, Cukjati et al. (2001) arranged in order of decreasing prediction capability, prognostic factors as follows:
- Wound size
- Patient's age
- Elapsed time from wound appearance to the beginning of the treatment
- Width-to-length ratio
- Location and type of treatment.

The chronic wounds that were included were greater than 3 months in a deteriorating or static phase included:
- Venous leg ulcers
- Trauma wounds
- Pressure ulcers
- Arterial ulcers

### Study objectives

To evaluate the effectiveness of Cool₂O Plus in non-healing chronic wounds of longer duration than 3 months. The parameters being measured:
- pain levels during wear time
- rate of healing
- ease of use
- ease of removal
- absorption capacity
- debridement of wounds

Study questions included:
1. During wear time, on application and removal, does the patient experience more or less pain, as measured by the 1-10 Verbal Descriptor Pain Scale (Nagata et al 1996), than with previous treatments?
2. Is the dressing easy to handle, apply and remove?
3. Are patients generally more or less satisfied with the study dressing regime?
4. Does the wound show signs of healing?
5. Does the dressing absorb without reflecting wound fluid back on to the surrounding skin?
6. Does the necrotic tissue / slough, debride with Cool₂O Plus?

### Subject selection

Patients who met the inclusion criteria were recruited from nursing homes, primary care trusts and personal referral.

### Inclusion criteria:

1. Signed informed consent
2. Adult patients over the age of 18 years
3. Patients with non-healing wounds of > 3 months duration
4. Patients able to demonstrate understanding through verbalization and performance, information about the study and the study dressing
5. Patients able to articulate information about their leg ulcer/pressure ulcer management

### Exclusion criteria:

1. Patients who in the judgement of the nurse were not appropriate for the study
2. Patients refusing to take part in the evaluation
3. Patients who have existing neurological disorders that would alter pain perception (i.e. Guillain-Barre syndrome, multiple sclerosis and myasthenia gravis)
4. Patients with pre-existing wound infection (confirmed by presence of cellulitis, positive wound swab) or other unrelated pain conditions
5. Patients with uncontrolled diabetes
6. Patients who are active alcohol and/or drug abusers
7. Patients currently taking immunosuppressants or any medication that would impair/influence wound healing.
8. Patients with a known sensitivity or allergy to the dressing
9. Patients who are moribund

### Patient assignment method

Prospective patients were assessed by the nurse for eligibility and informed consent was obtained prior to inclusion.

### Clinical examinations

Clinical examinations were limited to the following:
- Subjective data regarding general wound pain, especially when the dressing is in place, being applied and removed. The 1-10 Verbal Descriptor Pain Scale was used to measure the level of wound pain.
- Objective skin and wound assessments: the nurse conducted these assessments during the entry visit, weekly and on exit from the study
- Subjective information on the amount of exudate, based on the researcher's experience and the number of dressing changes and whether the number can be reduced
- Photographs were used to record the required evidence of wound changes

### Frequency and duration of the study

Patients were to be treated for a maximum of 3 weeks with subjective data and skin assessments to be evaluated on entry, weekly for 3 weeks and on exit from the study. At the midpoint of the trial it was decided to extend the trial to include a longer period of assessment.

### Results

The results are presented in case study format, with the result for each individual patient included. However, for the overall healing of the 8 wounds:
- On average, 58% of all wounds debrided
- Pain was an average of 3.75 on entry to the trial and 1.25 at exit. A reduction of pain by 66% in all painful wounds
- Healing rate was an average of 33% closure

### Case Study 1

Mr PW was a 78 year old gentleman with multiple medical problems. He had been extremely ill and was undoubtedly in a dying state. While in hospital he had developed some extremely large pressure ulcers. He was then transferred to a nursing home for full care.

The heel ulcer was extremely deep and there was dead bone loose in the wound. The wound was malodorous to such an extent that it could be detected throughout the corridor of the nursing home and the nurses were having difficulties with dressing the wound because of the smell. The wound was undermined by 2cms around the diameter of the wound.

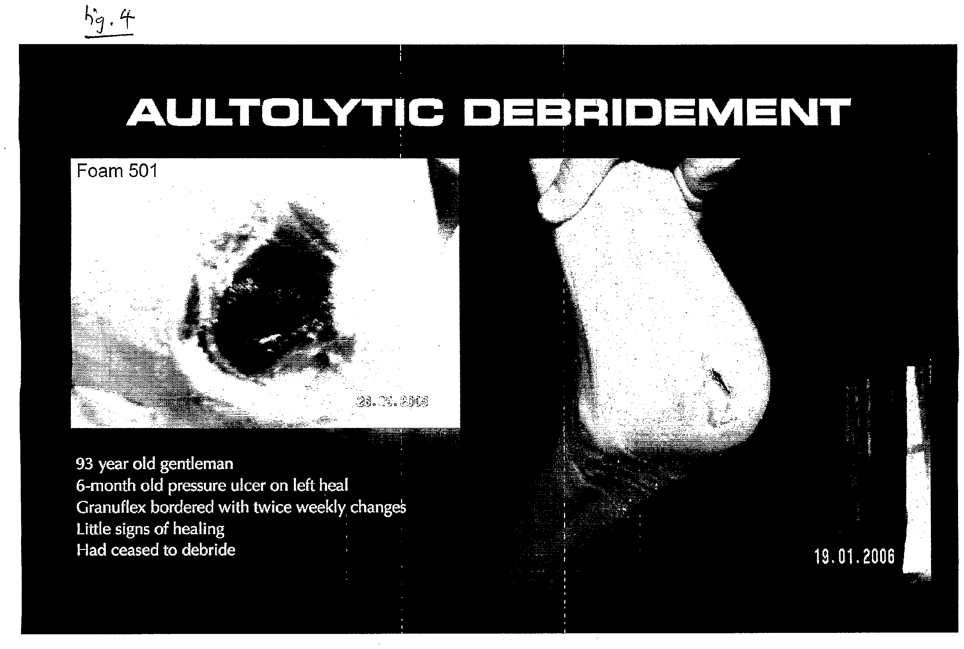

Cool₂O Plus was used to debride the wound and was applied on 11^{th} June 2005. The dressing absorbed the wound fluid (figure 4) and kept the wound bed moist. The secondary dressings were a heel shaped foam dressing (which had been used prior to this case study) and a bandage.

On the 18^{th} of June (7 days of treatment) the wound had begun to debride with good granulation tissue apparent.

By 22nd June the wound had almost fully debrided and there was no longer dead bone within the tissue. The undermining was almost closed.

On the 29th June, a clinical infection was noted with cellulites ascending the leg, and antibiotics were commenced for 5 days.

The infection resolved and the wound returned to a healing state. On 15th July (27 days of treatment) the wound was fully debrided centrally with a small amount of necrotic tissue in the margins, attached to the undermined section of the wound. The wound was filled with granulation tissue and was no longer malodorous.

The pain was at level zero at the beginning of the trial and at level zero at the end. The dressing was found to be easily removed and applied and it formed to the shape of the heel without difficulty. Both the nurse and the patient were satisfied with the performance of Cool₂O Plus.

The wound healed by 30% over a period of four weeks, the skin remained free of maceration and the necrotic tissue debrided by 60%.

In this case study, Cool₂O Plus had been helpful in debriding the wound and provided and ideal wound healing environment.

Unfortunately, Mr PW died.

### Case Study 2

Mr PW was a 78 year old gentleman with multiple medical problems. He had been extremely ill and was undoubtedly in a dying state. While in hospital he had developed some extremely large pressure ulcers. He was then transferred to a nursing home for full care.

The leg ulcer was due to his legs pressing together without the protection of a pillow. The ulcer was extremely deep, necrotic and malodorous. The wound margins showed signs of further damage as the tissue had dark erythema.

Cool₂O Plus was used to debride the wound. The dressing kept the wound bed moist. The secondary dressings were a pad and a light bandage.

After 7 days of treatment, the wound had begun to debride. On the eleventh day the necrotic tissue was fully rehydrated and was lifting away from the wound.

After 21 days of treatment the wound was fully debrided and the wound was filled with granulation tissue and was no longer malodorous.

After 34 days, the wound was fully debrided, free from infection, with granulation beginning to fill the wound.

The pain was at level 4 at the beginning of the trial and at level zero at the end. The dressing was found to be easily removed and applied with no associated pain. Both the nurses and the patient were satisfied with the performance of Cool₂O plus.

The wound healed by 10% over a period of four weeks, the skin remained free of maceration and dressing changes were reduced from daily to twice weekly. The necrotic tissue debrided by 100%.

In this case study Cool₂O Plus was helpful in debriding the wound and provided an ideal wound healing environment. Unfortunately Mr PW died.

### Case Study 3

Mrs OH is an 83 year old who is a resident in a nursing home. Her medical history includes epilepsy and she has elderly dementia.

Mrs OH was orphaned at 14 and taken in by an elderly childless couple. In her adult life she was a secretary and, eventually, a proprietor of a guest house. In her spare time she loved sunbathing and walking and spent her winters in Spain.

The nursing home had been her home for two years and her dementia tended to keep her isolated from other people although, occasionally, she had a spark of contact between her and the nurses.

Mrs OH had arterial insufficiency in her lower limbs and a decision had been made not to treat her as the ABPI was only just below the 'norm' ABPI of 0.8 and her dementia meant that she would not understand why the procedure was being carried out.

Due to the poor supply to her lower limb, the left leg, had developed an ulcer just above the lateral Malleolus, that was necrotic and had been intractable for 7 months.

Cool₂O Plus was applied to the ulcer.

After 9 days of treatment, the necrotic tissue had begun to lift and the edges of the wound had a cleaner appearance.

After 16 days of treatment, the necrotic tissue had lifted and debrided and tendon had become exposed. The wound had some depth to it and was granulating, but the granulation tissue was not of a good quality.

After 24 days of treatment, the wound was filled with good quality granulation which was level to the surface and the tendon was almost covered.

After 53 days of treatment, the wound had fully debrided and there were signs of healthy tissue and healing. The tendon had also debrided.

Mrs OH was unable to express her pain levels using numbers, but she appeared to experience less pain. There was no difficulty with removal of the dressing. The wound healed by 60%, the skin was clear without maceration and the necrotic tissue debrided by 100%.

Cool₂O Plus was helpful in debriding the wound and provided an ideal wound healing environment. This wound would have gone on to heal, but the arterial insufficiency was not supportive of healing and the area continued to break down.

Mrs OH required an amputation but this was not possible due to her frail condition. It was therefore decided to discontinue the case study.

### Case Study 4

Mrs GT was a 94 year old lady suffering from Alzheimer's disease. She lived in a nursing home. While in hospital she had developed a pressure ulcer over her right heel.

Cool₂O Plus was used to debride the wound. The dressing kept the wound bed moist. The secondary dressings were a pad and a light bandage.

After 39 days, the wound was fully debrided, free from infection, with granulation beginning to fill the wound. The wound margins were healthy and there was every evidence this wound would continue to healing.

The pain was zero and the beginning of the trial and zero at the end. The dressing was easily removed and easily applied, and it formed to the shape of the heel without problem. Both the nurses and the patient were satisfied with the performance of Cool₂O Plus. The wound had granulation tissue of approximately 80% which indicates healing. The skin remained free of maceration and the dressing was changed twice weekly, reduced from daily. The necrotic tissue debrided by 80%.

Cool₂O Plus was helpful in debriding the wound and provided an ideal wound healing environment. Unfortunately, Mrs GT died before completion of healing.

### Case Study 5

Mrs EW had a small painful wound on the lateral aspect of her left leg. Trauma had started the wound but arterial disease prevented it from healing. The wound base was pale and the pain would wake her at 2am. Both of these factors indicate arterial disease.

Cool₂O Plus was applied. The pain was immediately reduced and the dressing absorbed the fluid from the wound.

Within 5 days, the wound had begun to debride and was slightly cleaner. Certainly the pain was reduced.

The pain was 8 at the beginning of the trial and zero at the end. The dressing was easily removed and easily applied. Both the nurses and the patient were satisfied with the performance of Cool 2O Plus. The wound showed slight signs of healing but it was not possible to quantify the extent. The skin remained free of maceration and the dressing was changed twice weekly, reduced from 3 times weekly. The necrotic tissue debrided by 10%.

Cool₂O Plus was helpful in debriding the wound and provided an ideal wound healing environment. Unfortunately, Mrs EW was admitted to hospital and was discontinued from the trial.

### Case Study 6

Mrs ER is an 85 year old lady living in a nursing home. She is severely disabled and chair bound.

Mrs ER's wounds were multiple, oozing green exudate and painful. Cool₂O Plus was applied.

One week later, the skin was free from dead cells and dried exudate. The wounds were granulating and the skin appeared healthier.

The pain was rated at level 7 at the beginning of the trial and 5 at the end. The dressings were easily removed and easily applied. Both the nurses and the patient were satisfied with the performance of Cool₂O Plus. The wound had granulation tissue of approximately 80% which indicates healing. The skin remained free of maceration and the dressing was changed twice weekly, reduced from daily. The necrotic tissue debrided by 100%.

The wound bed was thus prepared for healing.

### Case Studies 7 and 8

Miss EB has a large venous leg ulcer on her left leg, that almost circumvents the ankle. There is a large open area on the medical aspect and a small area on the lateral aspect.

Miss EB is a difficult lady as she insists on making all decisions on wound care for herself. At the start of the trial, she insisted on having 3 weeks of Iodoflex and 3 weeks of Aquacel. This has been happening for several years with little healing occurring in the wound.

Exudate was also a problem and Miss EB had layers of gauze and pads plus Gamgee over the wound site to absorb the fluid.

She required some persuading to change treatment but did see that having the same treatment for years meant that it was no longer working.

There are many patients like Miss EB, with a determination to be admired. This determination means that they often will not like the dressing that is used because it is being used almost against their will. However, Miss EB had no pain in her leg for the first time for many months and this led her to be completely concordant with treatment.

Her wound continues to do well, with reduced potential for pain. The exudate loss is reduced and the number of pads has also been reduced accordingly. She is now committed to Cool₂O Plus in the way she had been committed to other dressings.

The pain was rated at level 8 at the beginning of the trial and zero at the end. The dressing was easily removed and easily applied and able to be cut to the shape of the wound. Both the nurses and the patient were satisfied with the performance of Cool₂O Plus. The wound showed signs of healing. The skin remained free of maceration and the dressing was changed twice weekly, reduced from daily.

Cool₂O Plus was an ideal dressing for Miss EB, as her main problems had been pain and exudate loss. Pain was no longer an issue and exudate had reduced enough to reduce dressing changes and the number of pads that is required to soak the fluid was minimised.

### Discussion

Cool₂O Plus absorbs a large amount of exudate and very efficiently retains it. When the dressing is removed from the wound, it has swollen to several times its own size. However, it does break up into pieces of gel which can look unpleasant. It should be noted, however, that that is no different to many other dressings, for example Iodoflex which resembles mashed potato when it is removed.

The pain levels are certainly reduced with Cool₂O Plus, and that reduction is significant. Each person with a painful wound found a reduction in pain.

Healing depended on the type of wound that was being assessed. All wounds showed some signs of healing, to greater or lesser degree. It is evident that Cool₂O Plus does provide an ideal wound healing environment.

### Conclusion

The Cool₂O Plus is able to provide a moist, occlusive environment that reduces pain in painful wounds. It is particularly suitable for patients with painful wounds, those with exuding wounds that have a potential for maceration and those wounds requiring debridement.

It should be understood that this dressing covers the entire wound healing continuum; an unusual property in dressings.

### Example 21

### Autolytic Debridement and Slough Management

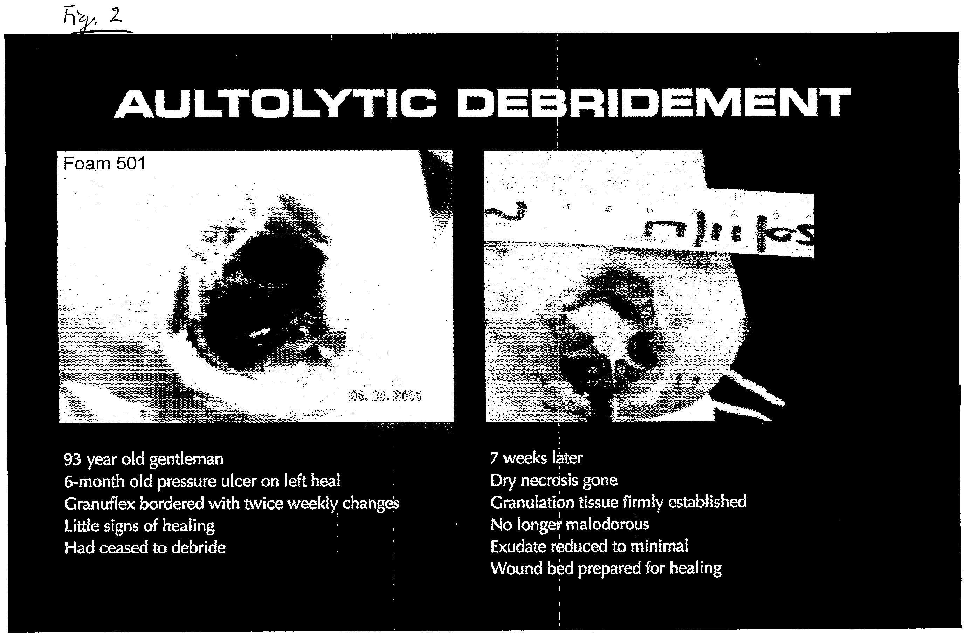

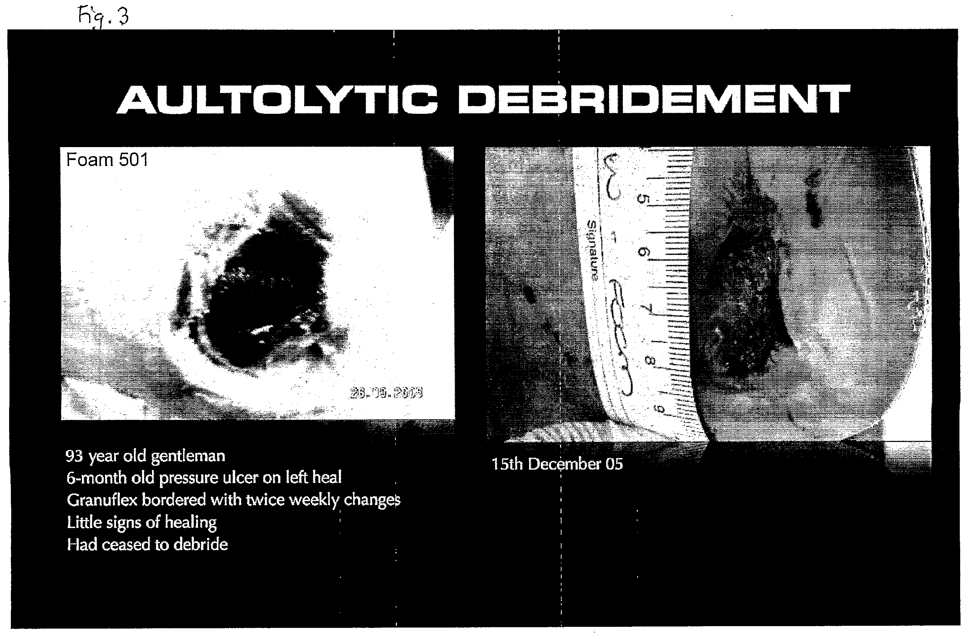

Figures 2 to 4 of the accompanying drawings show the stages of treatment of a chronic (6-month old) pressure ulcer on the left heal of a 93-year old gentleman, particularly to illustrate the effective autolytic debridement and slough management achieved by the hydrogel dressing according to the present invention.

Prior unsuccessful treatment had employed the conventional dressing Bordered Granuflex (available from ConvaTec Limited), which consists of a thin polyurethane foam sheet bonded onto a semipermeable polyurethane film which is impermeable to exudate and micro-organisms. The surface of the dressing to be placed in contact with the wound is coated with a hydrocolloid mass, which is a cross-linked adhesive mass containing a dispersion of gelatin, pectin and carboxymethylcellulose together with other polymers and adhesives forming a fexible wafer. When the dressing comes into contact with wound exudate, the polysaccharides and other polymers absorb water and swell, forming a gel. The adhesive foam with a thin layer of hydrocolloid extends beyond the central hydrocolloid mass to provide a border with low profile edges for extra security in awkward areas.

The hydrogel used was the hydrogel prepared in Example 8, coated onto an open-cell polyurethane foam at a hydrogel:polyurethane weight ratio of 2:1, and designated "Foam 501" for study purposes.

The progression of the treatment from the start date of 26 September 2005 (left-hand photograph in Figures 2, 3 and 4) up to a final date of 19 January 2006 is shown in Figures 2, 3 and 4. The remarkable healing of the chronic wound, and the highly effective autolytic debridement induced by treatment according to the present invention, is clear.

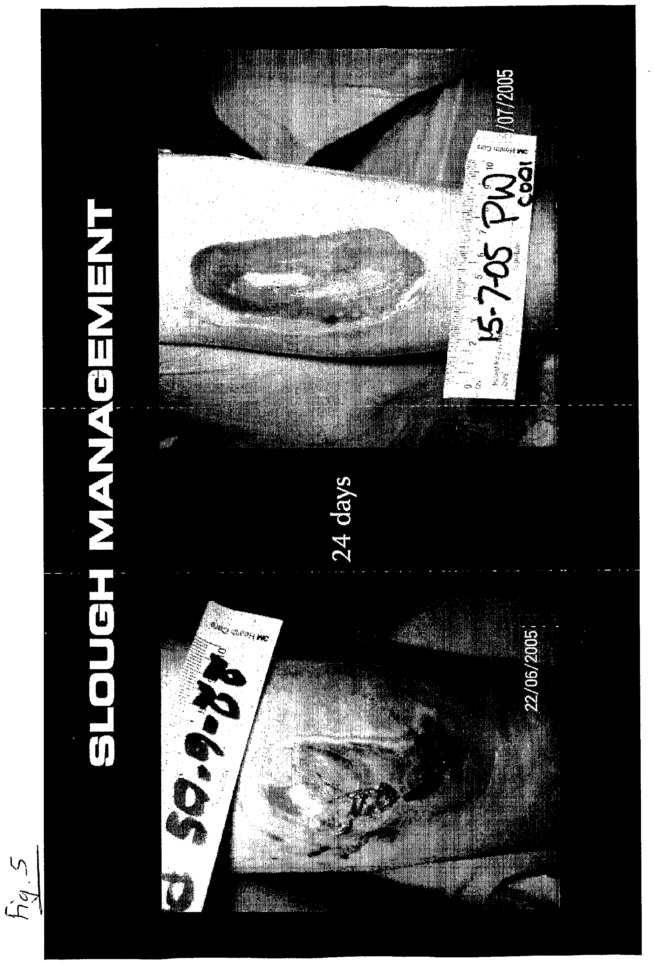

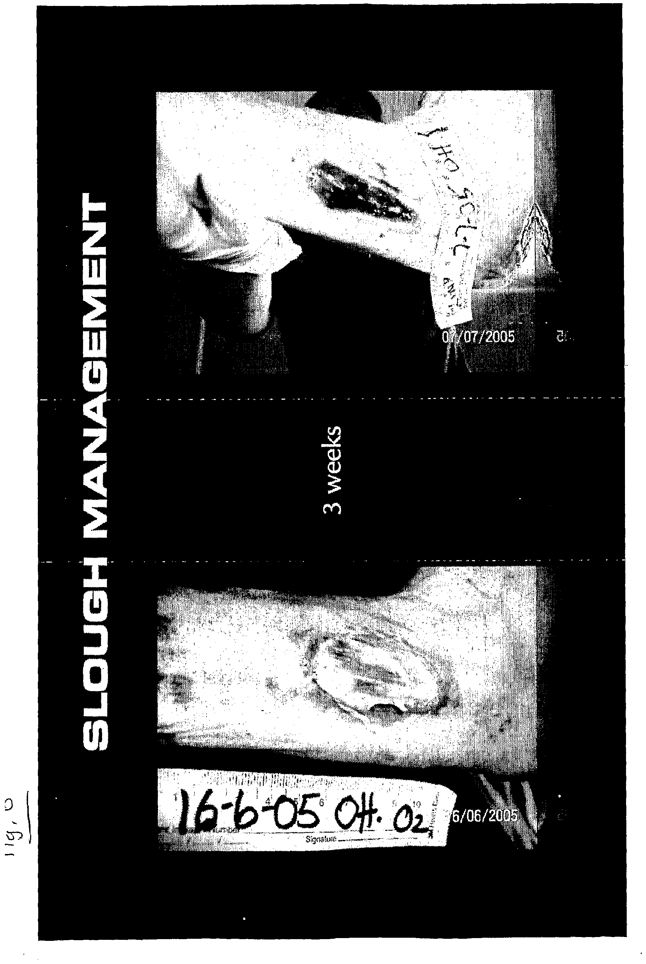

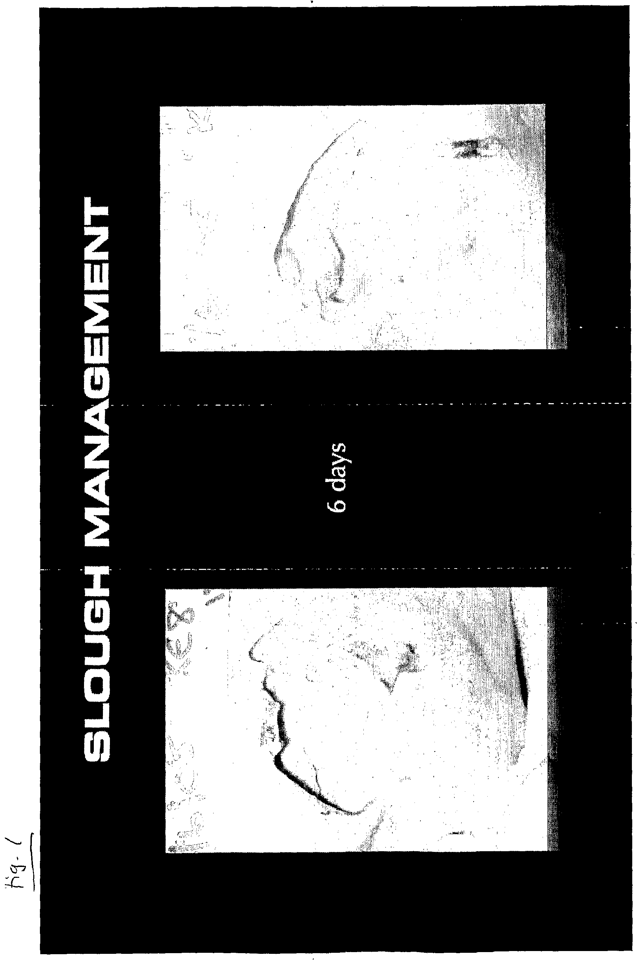

Figures 5 to 7 illustrate slough management achieved using the present invention. As shown, over periods of days or a few weeks (Figure 5 - 24 days; Figure 6 - 3 weeks; Figure 7 - 6 days) the sloughing associated with a chonic wound was markedly reduced using a dressing containing the hydrogel of Example 8.

### Example 22

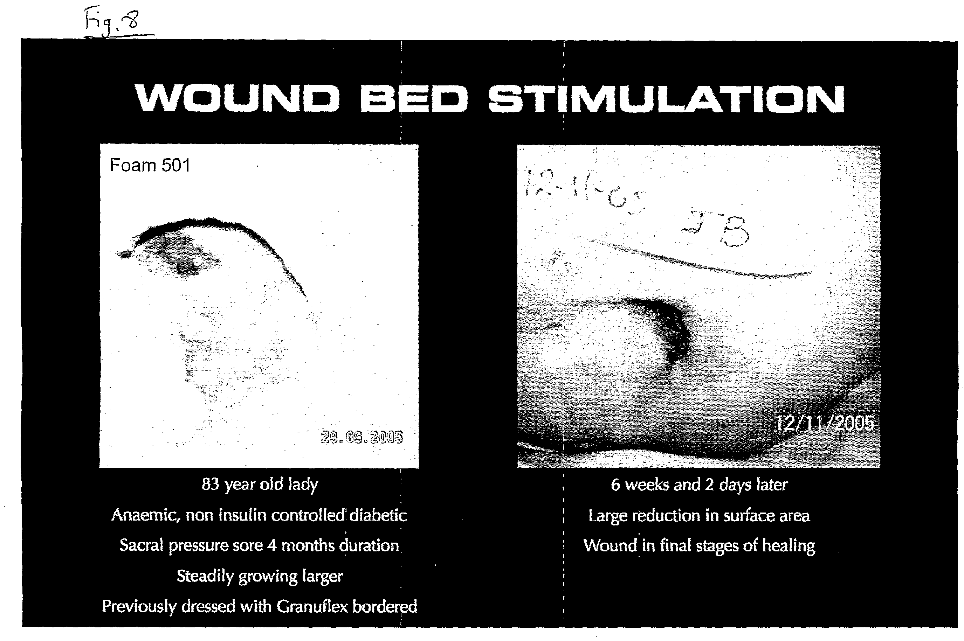

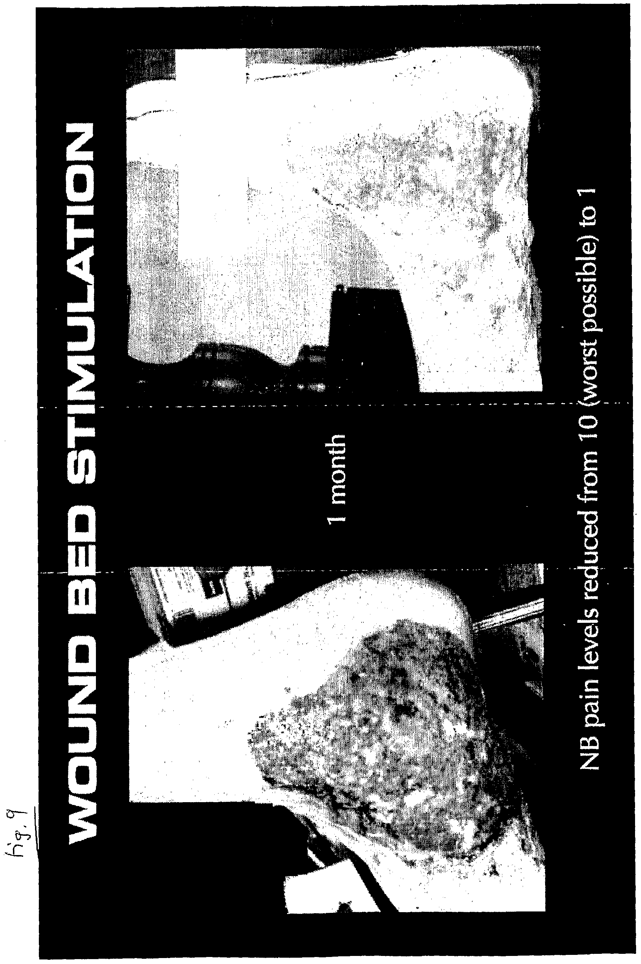

The dressing Foam 501 was used to treat the chronic skin wounds shown and described in Figures 8 and 9 of the accompanying drawings. The wound in the case of Figure 8 (left hand photograph, showing the start of treatment according to the present invention) was a 4-month old chronic sacral pressure sore, steadily growing larger, previously dressed unsuccessfully with Bordered Granuflex. The patient was an 83-year old lady, who was an anaemic, non-insulin-controlled diabetic.

The right hand photograph of Figure 8 shows the condition of the wound after treatment according to the present invention for 6 weeks and 2 days. There is a large reduction in the surface area of the wound and the wound is in the final stages of healing.

Figure 9 shows a chronic ankle wound at the start (left hand photograph) of treatment and after one month of treatment (right hand photograph) according to the present invention, using the dressing Foam 501. In this case, it was reported that the pain levels associated with the wound reduced from 10 on the standard scale employed in wound care (the worst possible level) to 1, nearly at zero level (0).

### Example 23

Studies were undertaken to compare the effect of the hydrogels of the present invention as hydraters and conditioners of skin in patients having chronic wounds. Roughness and scaliness of the skin is a condition frequently associated with chronic skin wounds.

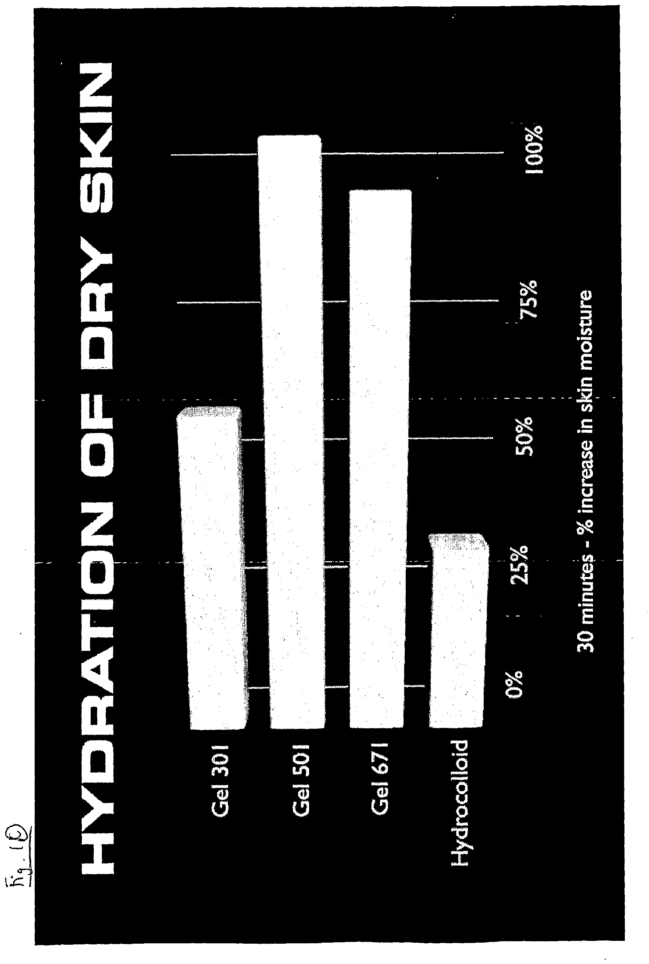

Figure 10 of the accompanying drawings shows the results of the experiment to measure the hydration effects of three hydrogels according to the present invention, namely "Gel 301", which is the hydrogel obtained in Example 1, "Gel 501", which is the hydrogel obtained in Example 8, and "Gel 671", which is the hydrogel obtained in Example 2. The comparison material is Granuflex, a conventional commercial hydrocolloid used in wound care.

The test involved applying the materials to human skin for a period of 30 minutes and measuring the change in skin moisture using a Courage & Khazaka Electronics CM825 comeometer.

As shown by Figure 10, the hydrogels according to the present disclosure hydrate the skin substantially more effectively over 30 minutes than the conventional hydrocolloid.

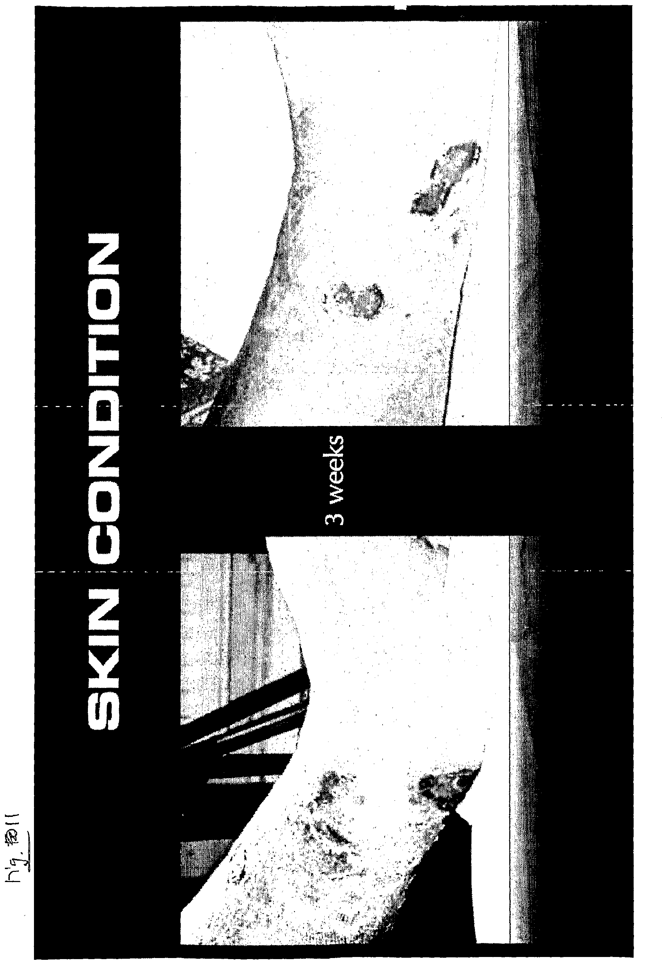

Figure 11 of the accompanying drawings shows the improvement in skin condition achieved by the treatment of the present invention over a period of 3 weeks. The starting condition is shown in the left-hand photograph, showing very scaly skin, which substantially improved its condition after the treatment, as shown in the right-hand photograph.

### Example 24

Tryptone soya agar plates were inoculated from stock cultures of the following bacteria:

| | |
|---|---|
| *Pseudomonas aeruginosa* | NCIMB 8626 |
| *Staphylococcus aureus* | NCTC 10788 |
| *Escherichia coli* | NCIMB 8545 |

These cultures were incubated at 30-35°C for 18-24 hours.

A sabouraud dextrose agar plate was inoculated from the stock culture of *Candida albicans* NCPF 3179 and incubated at 20-25°C for 48 hours.

Three sabouraud dextrose agar plates were inoculated from the stock culture of *Aspergillus niger* IMI 149007 and incubated at 20-25°C for 7 days.

The bacteria and *C*. *albicans* ("yeast") were harvested using 0.1% peptone water containing 0.9% sodium chloride to wash the surface growth from each plate into separate sterile universal bottles.

The resultant suspension were further diluted with the same liquid to reduce the count to approximately 1x10⁸ cfu/ml.

*Aspergillus niger* ("mould") was harvested in a similar manner with 0.9% saline containing 0.05% Polysorbate 80.

The suspensions were used immediately.

Thirty pieces of a Cool₂O Plus gel sample were aseptically cut into 2cm². The samples were inoculated with 0.1 ml of 108 cfu/ml of appropriate inoculum. Six with *C*. *albicans,* six with *A. niger,* six with *P. aeruginosa,* six with *S. aureus* and six with *E. coli.*

The product squares were then placed separately into sterile universals, to be used at the specific timepoints. The same volume of inoculum was simultaneously introduced into separate equivalent quantities of 0.1% peptone water containing 0.9% sodium chloride (bacteria and yeast) and saline/polysorbate 80 (mould) to be used as controls.

The inoculated product was in each case stored in the dark at 20-25°C.

At 0 hrs five universals, one containing each organism, were removed from the incubator and 10 mls of 0.1% peptone water containing 0.9% sodium chloride was introduced into each sample. This was repeated at 24 and 48 hours, 7, 14 and 28 days. The product was left to stand for 30 minutes and then vortexed for 1 minute.

1 ml of product was added to 9 ml of 0.1% peptone water containing the following as preservative inactivating agents (all percentages by weight):

| | |
|---|---|
| Polysorbate | 80 |
| Lecithin | 0.5% |
| Triton X100 | 1.0% |

### Sodium thiosulphate

The control preparations were similarly sampled at 0 hours to determine the viable counts of the cultures used and to confirm the suitability of the media used for their growth. Further dilutions were made as necessary in 0.1% peptone water containing 0.9% sodium chloride. 1 ml aliquots of all dilutions were incorporated in duplicate pour plates of the appropriate cooled molten agar.

The pour plates were incubated at 30-35°C for 3 days for the bacteria and at 20-25oC for 5 days for the yeast and mould.

After incubation the number of colonies on each plate were counted and, taking the dilution factor into account, the number of cfu/ml of product calculated. These figures are listed in the tables below.

The suspensions of the test organisms were further diluted with 0.1% peptone water containing 0.9% sodium chloride to approximately 10³ cfu/ml.

Four Petri dishes were used for each organism and 0.1 ml of the relevant suspension added to each plate.

To the first set of plates 1 ml of product diluted 10-fold in recovery medium was added, to the second set 1 ml of product diluted 100-fold was added, to the third set 1 ml of product diluted 1000 fold was added and the fourth set acted as a control having no product in them.

The appropriate cooled molten agar was then added to the plates which were incubated as described above. The plates were then examined for growth and the number of colonies present recorded.

### Results

### Control count at 0 hour

| Organism | cfu/ml |
|---|---|
| *C. albicans* | 4.5 x 10⁵ |
| *A. niger* | 2.7 x 10⁵ |
| *P. aeruginosa* | 2.8 x 10⁵ |
| *S. aureus* | 4.9 x 10⁵ |
| *E. coli* | 1.8 x 10⁵ |

### Validation of recovery counts

Validation control count at 0 hour (control and three levels of dilution, namely 10⁻¹ dilution, 10⁻² dilution and 10⁻³ dilution).

| Organism | Control | 10⁻¹ | 10⁻² | 10⁻³ |
|---|---|---|---|---|
| *C. albicans* | 43 | 40 | 42 | 42 |
| *A. niger* | 23 | 23 | 23 | 24 |
| *P. aeruginosa* | 20 | 6 | 19 | 20 |
| *S. aureus* | 45 | 44 | 46 | 45 |
| *E. coli* | 16 | 16 | 15 | 16 |

The product did not return a count of greater than 80% of the validation control count at the 10⁻¹ dilution for *P. aeruginosa.* At 10⁻² dilution the count was in excess of 80%, therefore the product is said to inhibit growth of *P. aeruginosa* at 10⁻¹ dilution, but is valid at 10⁻² dilution. Taking into account the dilution factors, counts of <50 cfu/ml are therefore valid for this organism. No inhibition was noted on validation plates for the other organisms. Counts of <5 cfu/ml are therefore valid for these organisms.

### Recovery counts from test product

### Mean counts/ml sample after:

| | **0 hr** | **24 hr** | **48 hr** | **7 days** | **14 days** | **28 days** |
|---|---|---|---|---|---|---|
| *C. albicans* | 4.2x10⁵ | 6.6x10⁴ | 8.0x10⁴ | 4.1x10⁴ | 6.2x10³ | 4.9x10³ |
| *A. niger* | 2.4x10⁵ | 2.6x10⁴ | 9.6x10² | 6.5x10² | 1.1x10³ | 1.6x10³ |
| *P. aeruginosa* | 2.2x10⁵ | <50 | <50 | <50 | <50 | <50 |
| *S. aureus* | 4.0x10⁵ | 4.8x10² | <5 | <5 | <5 | <5 |
| *E. coli* | 1.4x10⁵ | <5 | <5 | <5 | <5 | <5 |

### Interpretation of the results

It was observed that C. *albicans* had a reduced count at 7 days of one log and by 4 days the count was 6.2x10³ cfu/ml.

A two log reduction was observed for *A. niger.* However, this increased slightly after 14 days. *P. aeruginosa* and *E. coli* showed no recovery from 24 hrs onwards and *S. aureus* showed no recovery from 48 hrs.

### Example 25

### Preservative Efficacy Challenge Testing

### Purpose

To demonstrate the ability of a product to withstand amicrobial insult which may occur during intended use. This method is based on inoculation of a product with microorganisms and monitoring microbial reduction over a specified period of time.

### Method

The method is essentially the same as used in Example 21. In this test 2 cm² of hydrogel dressing were innoculated with a known number of colony forming units (cfu's) per ml of appropriate innoculum, i.e. *Candida albicans, Aspergillus niger, Pseudomonas aeruginosa, Staphylococcus aureus* or *Escherichia coli.*

The innoculated samples were incubated for a period of time and then the microrganisms were recovered and plated on agar plates and then incubated to allow the organisms to grow. The number of cfu's recoverable from the hydrogel ("dressing") at time 0 and at 1,2, 7, 14 and 28 days were determined.

### Results

| Sample | Time (days) | | | | | |
|---|---|---|---|---|---|---|
| | 0 | 1 | 2 | 7 | 14 | 28 |
| *Candida albicans* | 370,000 | 48,000 | Less than 5 | Less than 5 | Less than 5 | Less than 5 |
| *Aspergillus niger* | 220,000 | 380 | 170 | Less than 5 | Less than 5 | Less than 5 |
| *Pseudomonas aeruginosa* | 250,000 | Less than 50 | Less than 50 | Less than 50 | Less than 50 | Less than 50 |
| *Staphylococcus aureus* | 390,000 | Less than 5 | Less than 5 | Less than 5 | Less than 5 | Less than 5 |
| *Escherichia coli* | 130,000 | Less than 5 | Less than 5 | Less than 5 | Less than 5 | Less than 5 |

### Conclusion

The results show that on contacting the hydrogel dressing all the bacteria are effectively killed within a day the fungus and yeast within 2 days. Once killed they remain so.

### Example 26

### Microbial Nutrient Uptake

In the studies reported in Example 25 above, wounds have been observed that were clearly colonised with *Pseudomonas aeruginosa*, which were green prior to application of the dressing. On removal of the dressing, the green colour was found to be integrated into the hydrogel.

Since it is highly unlikely that the large *Pseudomonas* bacteria are absorbed into the dressing, it is believed that but the Fe³⁺ siderophores secreted by the bacteria are responsible for the coloration uptake.

There is no reason to doubt that ionic nutrient uptake by the hydrogels is limited only to siderophores or the Fe³⁺ ions.

### Example 27

The following experiment was performed to determine the dynamic fluid handling properties of wound dressings according to the present disclosure.

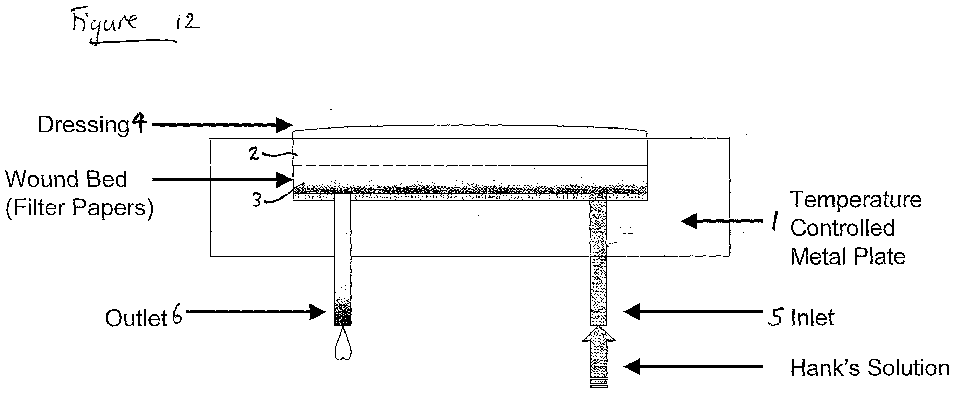

The apparatus is shown schematically in Figure 12 of the accompanying drawings, and comprises a temperature-controlled absorbency testing platform 1 defining a central depression adapted to hold two stacked filter papers 2, 3 and an overlying dressing 4 consisting of a Cool₂O Plus gel sample. The well receives Hanks' Balanced Salt Solution via a bottom inlet 5 and the Hanks' solution exits the central depression via bottom outlet 6 arranged slightly higher than the inlet 5.

In more detail, the equipment used in this experiment was as follows: Electronic top-pan balance with integral RS232 serial interface (a calibrated balance is preferable); Electronic logger and appropriate interface to capture data from the balance; Balance (4 figure); Absorbency testing platform and support table (As supplied by Surgical Materials Testing Laboratory, Bridgend, UK); Delivery system for test solution - Graseby 3100 Syringe Pump; 10cm x 10cm perforated mesh spreader plate and weight -(600g in total); Supply of 47mm diameter cellulosic absorbent pads,. (Millipore Catalogue Number AP1004700); Supply of waterproof impermeable adhesive tape (e.g. Sleek or similar); Fluid collection vessel with a small surface area; Hanks Balanced Salt Solution - Ref: Sigma H9269; Light machine oil or similar lightweight oil; Scissors; Tweezers; Gloves; Supply of 60ml syringes for syringe pump; Supply of 2.5ml sterile syringes for extracting fluid from absorbent pads at the end of the test; Supply of absorbent tissue; De-ionised water (for cleaning); Small Petri dishes (or similar containers).

All the equipment to be used must be scrupulously clean. The tweezers are used to handle the absorbent pads and the dressing. If equipment or materials are to be handled, clean gloves must be worn.

The balance was first levelled and the platform adjusted if necessary to slope upwards at 2° from the inlet 5 towards the outlet 6.

The experimental method was then as follows:
1. Place the balance on the bottom tier of the support table and level.
2. Turn the balance on and leave for at least 30 minutes to warm up and settle.
3. Zero the balance.
4. Connect the datalogger to the balance.
5. Set up the datalogger to record for the required time period at a rate of at least one reading every five minutes.
6. Place a suitable collecting vessel on the top pan balance; the collecting vessel should have a small amount of test solution covered with a layer of light machine oil on the surface to prevent evaporation loss.
7. Fill the fluid delivery system with Hanks Balanced Salt Solution, ensuring that there is sufficient fluid in the system to complete the test you are doing and remove any air bubbles from the system
8. Set the fluid delivery system to the required flow rate, connect to the platform and run the system until fluid starts to fill the channel in the centre of the depression in the platform.
9. Stop the fluid delivery system and remove the excess fluid from the channel using absorbent tissue.
10. Separately weigh two dry absorbent pads and record the weight of each.
11. Place the two pads in the central depression of the testing platform, noting which pad is placed on the bottom of the depression (in contact with the fluid), and which pad is placed on the top (in contact with the dressing).
12. Weigh the dressing to be used (plus any liners) and record the weight.
13. Using the fluid delivery system, purge the system with 2ml fluid twice, ensuring that the pads are fully wetted.
14. Dry the outlet tube to remove any fluid that may not have fully dropped over.
15. Remove the liners from the dressing to be tested and place the dressing centrally over the pads. Record the weight of the removed liners.
16. Use a plastic impermeable waterproof adhesive tape to seal down the dressing over the central depression.
17. Place the perforated spreader plate (10cm x 10cm) on top of the dressing and place the weight on top (total weight 600g).
18. Zero/reset the display on the fluid delivery system.
19. Tare the balance and then start the datalogger and test solution delivery system and run them for the required time period. Make a note of the start and finish time.
20. At the end of the testing period, stop the datalogger and the test solution delivery system, read the display on the delivery system (to confirm the amount of fluid delivered), and download the datalogger.
21. Remove the spreader plate and weight.
22. Carefully remove the tape from the dressing and record the weight of the dressing.
23. Remove each absorbent pad into a clean container, making a note of the top and bottom pad and record the weights of each pad.
24. Using tweezers and/or gloved hands, roll each pad and put into a clean, sterile, 2.5 ml syringe (again noting whether the top or bottom pad).
25. Squeeze the pad to extract the fluid (a minimum of 0.2ml is required) and store in a clean, sealable sample vial until testing.
26. Clean the central depression on the testing platform with purified water before starting another test.

The extracted fluid was then selectively analysed for sodium, potassium and calcium content as follows.

Each fluid sample was transferred to a 0.5ml sample cup. The system was calibrated according to the calibration in Section 4, Manual Update No. 7, for the Beckman SYNCHRON EL-*ISE*® Electrolyte System, for the following set points:-

| | |
|---|---|
| Sodium | 100 - 150mmol/l |
| Potassium | 4 - 10mmol/l |
| Calcium | 0.5 - 2.5mmol/l |

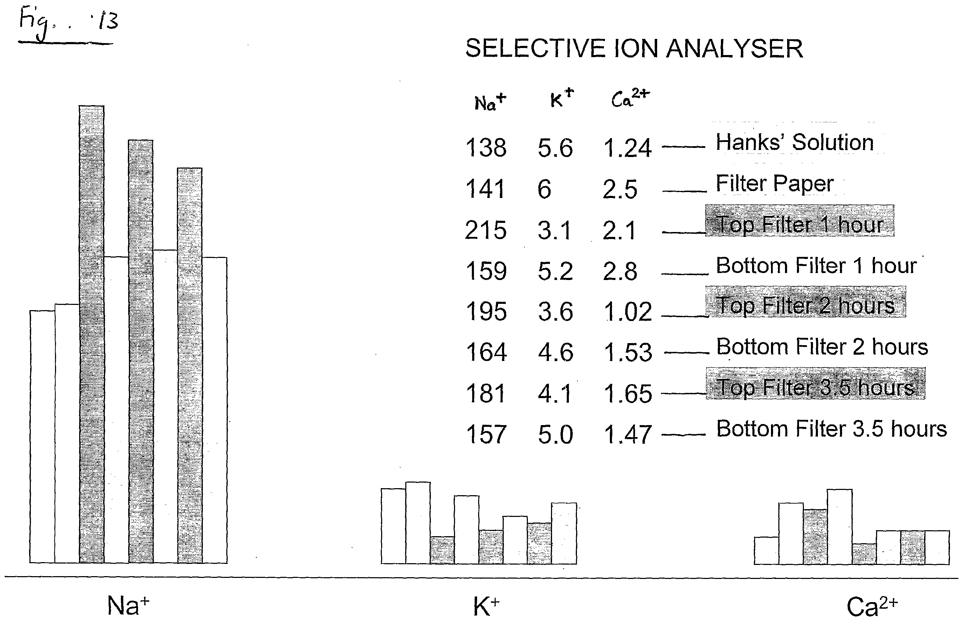

The samples were run as stated in the Manual for the Beckman SYNCHRON EL*-ISE*^{®} Electrolyte System. The results are shown in Figure 13 of the accompanying drawings. The concentration (mmol/l) of the individual ions sodium, potassium and calcium are shown (top to bottom of the Figure) for the starting Hanks' solution, the starting filter papers after initial soaking, and the top and bottom filter papers individually after 1, 2 and 3.5 hours of passage of Hanks' solution through the apparatus with the dressing in place. The same data is also represented graphically, in the same order of the bars left-to-right as the top-to-bottom order of the data figures.

It is seen that the dressing selectively increases the sodium ion concentration in the top filter paper (in contact with the dressing), representing the upper part of a wound, in comparison with the bottom filter paper, representing the wound bed, and selectively decreases the potassium ion concentration in the top filter paper in comparison with the bottom filter paper, with relatively little effect on the calcium ion concentration in either.

The effects were greatest about one hour after start, and reduced at 2 and 3.5 hours after start, but were appreciable even at those later times.

We believe that this effect is due to the hydrogel dressing according to the invention disclosure taking up sodium ions from the Hanks' solution slower than it takes up water from the Hanks' solution, and taking up potassium ions from the Hanks' solution faster than it takes up water from the Hanks' solution. It is believed that this effect arises correspondingly in the *in vivo* situation of the dressing on a wound, except that the Hanks' solution is replaced in that situation by wound exudate. As discussed above, it is believed that the effect is a consequence of the Donnan exclusion mechanism deriving from the large negative electrostatic charge of the hydrogel acting on the chloride anions of the (saline) wound fluid, which consequently restricts the uptake of the sodium counterion from the wound fluid.

### Example 28

Example 27 was repeated using a dressing made using a cross-linked copolymer of NaAMPS and SPAK according to Example 4.

The results (mean and standard deviation (SD)) of the ion analysis from the top and bottom filter papers are shown in the Table below, units mmol/l measured at time = 0, 0.5 hours, 1.00 hours, 2.00 hours and 3.50 hours:

| **Time (hours)** | **Mean** | **SD** |
|---|---|---|
| **0.00 Sodium top** | 144.00 | |
| **0.50Na** | 200.62 | 2.86 |
| **1.00Na** | 230.00 | 0.00 |
| **2.00Na** | 220.42 | 9.63 |
| **3.50Na** | 217.40 | 1.37 |
| | | |
| **0.00 Potassium top** | 5.95 | |
| **0.50K** | 2.86 | 0.09 |
| **1.00K** | 2.58 | 0.31 |
| **2.00K** | 2.66 | 0.26 |
| **3.50K** | 2.66 | 0.15 |
| | | |
| **0.00 Calcium top** | 1.80 | |
| **0.50Ca** | 0.97 | 0.14 |
| **1.00Ca** | 1.09 | 0.13 |
| **2.00Ca** | 0.89 | 0.08 |
| **3.50Ca** | 0.74 | 0.01 |
| | | |
| **0.00 Sodium bottom** | 144.00 | |
| **0.50Na** | 147.34 | 0.93 |
| **1.00Na** | 156.24 | 3.21 |
| **2.00Na** | 160.58 | 8.93 |
| **3.50Na** | 181.08 | 12.72 |
| | | |
| **0.00 Potassium bottom** | 5.93 | |
| **0.50K** | 5.44 | 0.13 |
| **1.00K** | 5.04 | 0.36 |
| **2.00K** | 5.02 | 0.58 |
| **3.50K** | 3.40 | 0.60 |
| | | |
| **0.00 Calcium bottom** | 1.72 | |
| **0.50Ca** | 1.74 | 0.17 |
| **1.00Ca** | 1.42 | 0.19 |
| **2.00Ca** | 1.37 | 0.04 |
| **3.50Ca** | 1.00 | 0.09 |

### Example 29

Example 27 was repeated using a dressing made using a cross-linked copolymer of NaAMPS and SPAK according to the following details:
Pre-gel: 70 parts by weight of 58% aqueous solution of the sodium salt of acrylamidomethylpropanesulphonic acid (Na AMPS, LZ2405 Lubrizol), 30 parts glycerol, 0.16 parts acrylic acid (3-sulphopropyl) ester potassium salt, commonly known as SPA or SPAK (SPA or SPAK is available commercially in the form of a pure solid from Raschig and 0.14 parts of a 1 to 10 (by weight) mixture of Daracure 1173 photoinitiator (Ciba Speciality Chemicals) and IRR280 cross-linker (PEG400 diacrylate, UCB Chemicals).

The results (mean and standard deviation (SD)) of the ion analysis from the top and bottom filter papers are shown in the Table below, units mmol/l measured at time = 0, 0.5 hours, 1.00 hours, 2.00 hours and 3.50 hours:

| **Time (hours)** | **Mean** | **SD** |
|---|---|---|
| **0.00 Sodium top** | **144.00** | |
| **0.50Na** | **224.50** | **9.53** |
| **1.00Na** | **230.00** | **0.00** |
| **2.00Na** | **230.00** | **0.00** |
| **3.50Na** | **230.00** | **0.00** |
| | | |
| **0.00 Potassium top** | **5.95** | |
| **0.50K** | **4.50** | **0.36** |
| **1.00K** | **4.07** | **0.15** |
| **2.00K** | **4.30** | **0.26** |
| **3.50K** | **3.87** | **0.15** |
| | | |
| **0.00 Calcium top** | **1.80** | |
| **0.50Ca** | **1.50** | **0.14** |
| **1.00Ca** | **1.44** | **0.16** |
| **2.00Ca** | **1.28** | **0.07** |
| **3.50Ca** | **1.10** | **0.03** |
| | | |
| **0.00 Sodium bottom** | **144.00** | |
| **0.50Na** | **162.83** | **6.14** |
| **1.00Na** | **180.73** | **13.75** |
| **2.00Na** | **200.13** | **17.65** |
| **3.50Na** | **222.93** | **12.24** |
| | | |
| **0.00 Potassium bottom** | **5.92** | |
| **0.50K** | **5.57** | **0.31** |
| **1.00K** | **5.63** | **0.21** |
| **2.00K** | **5.70** | **0.36** |
| **3.50K** | **5.00** | **0.30** |
| | | |
| **0.00 Calcium bottom** | **1.72** | |
| **0.50Ca** | **1.65** | **0.19** |
| **1.00Ca** | **1.51** | **0.14** |
| **2.00Ca** | **1.44** | **0.12** |
| **3.50Ca** | **1.32** | **0.17** |

### Example 30

Example 27 was repeated using a dressing made using a cross-linked copolymer of NaAMPS and SPAK according to the following details:
Pre-gel: 70 parts by weight of 58% aqueous solution of the sodium salt of acrylamidomethylpropanesulphonic acid (Na AMPS, LZ2405 Lubrizol), 30 parts glycerol, 0.5 parts acrylic acid (Aldrich) and 0.14 parts of a 1 to 10 (by weight) mixture of Daracure 1173 photoinitiator (Ciba Speciality Chemicals) and IRR280 cross-linker (PEG400 diacrylate, UCB Chemicals).

The results (mean and standard deviation (SD)) of the ion analysis from the top and bottom filter papers are shown in the Table below, units mmol/l measured at time = 0, 0.5 hours, 1.00 hours, 2.00 hours and 3.50 hours:

| **Time (hours)** | **Mean** | **SD 0.00** |
|---|---|---|
| **0.00 Na top paper** | **144.00** | |
| **0.50Na** | **230.00** | |
| **1.00 Na** | **224.73** | **9.12** |
| **2.00Na** | **225.63** | **7.56** |
| **3.50Na** | **226.10** | **6.75** |
| | | |
| **0.00 Potassium top** | **5.95** | |
| **0.50K** | **3.93** | **0.15** |
| **1.00K** | **4.00** | **0.66** |
| **2.00K** | **4.17** | **0.74** |
| **3.50K** | **4.27** | **0.64** |
| | | |
| **0.00 Calcium top** | **1.80** | |
| **0.50Ca** | **1.35** | **0.25** |
| **1.00 Ca** | **1.36** | **0.09** |
| **2.00 Ca** | **1.46** | **0.34** |
| **3.50 Ca** | **1.44** | **0.36** |
| | | |
| **0.00 Sodium bottom** | **144.00** | |
| **0.50Na** | **179.90** | **33.59** |
| **1.00Na** | **186.80** | **20.09** |
| **2.00Na** | **192.20** | **16.44** |
| **3.50Na** | **199.10** | **19.39** |
| | | |
| **0.00 Potassium bottom** | **5.92** | |
| **0.50K** | **5.37** | **0.42** |
| **1.00K** | **5.60** | **0.66** |
| **2.00K** | **5.50** | **0.75** |
| **3.50K** | **5.27** | **1.01** |
| | | |
| **0.00 Calcium bottom** | **1.72** | |
| **0.50Ca** | **1.54** | **0.21** |
| **1.00Ca** | **1.54** | **0.23** |
| **2.00Ca** | **1.51** | **0.29** |
| **3.50Ca** | **1.45** | **0.37** |

### Example 31

Example 27 was repeated using a dressing made using a cross-linked copolymer of NaAMPS and SPAK according to the following details:
Pre-gel: 70 parts by weight of 58% aqueous solution of the sodium salt of acrylamidomethylpropanesulphonic acid (Na AMPS, LZ2405 Lubrizol), 30 parts glycerol, 0.5 parts glucose (Aldrich) 0.14 parts of a 1 to 10 (by weight) mixture of Daracure 1173 photoinitiator (Ciba Speciality Chemicals) and IRR280 cross-linker (PEG400 diacrylate, UCB Chemicals).

The results (mean and standard deviation (SD)) of the ion analysis from the top and bottom filter papers are shown in the Table below, units mmol/l measured at time = 0, 0.5 hours, 1.00 hours, 2.00 hours and 3.50 hours:

| **Time (hours)** | **Mean** | **SD** |
|---|---|---|
| **0.00 Sodium top** | **144.00** | |
| **0.50Na** | **230.00** | **0.00** |
| **1.00 Na** | **230.00** | **0.00** |
| **2.00Na** | **230.00** | **0.00** |
| **3.50Na** | **230.00** | **0.00** |
| | | |
| **0.00 Potassium top** | **5.95** | |
| **0.50K** | **3.60** | **0.10** |
| **1.00K** | **3.73** | **0.40** |
| **2.00K** | **3.80** | **0.40** |
| **3.50K** | **3.40** | **0.30** |
| | | |
| **0.00 Calcium top** | **1.80** | |
| **0.50Ca** | **1.43** | **0.04** |
| **1.00Ca** | **1.38** | **0.10** |
| **2.00Ca** | **1.24** | **0.05** |
| **3.50Ca** | **1.15** | **0.04** |
| | | |
| **0.00 Sodium bottom** | **144.90** | |
| **0.50Na** | **162.27** | **4.44** |
| **1.00Na** | **180.13** | **13.61** |
| **2.00Na** | **206.30** | **8.86** |
| **3.50Na** | **217.17** | **13.61** |
| | | |
| **0.00 Potassium bottom** | **5.92** | |
| **0.50K** | **5.57** | **0.06** |
| **1.00K** | **5.07** | **0.35** |
| **2.00K** | **4.87** | **0.23** |
| **3.50K** | **4.53** | **0.21** |
| | | |
| **0.00 Calcium bottom** | **1.72** | |
| **0.50Ca** | **1.62** | **0.12** |
| **1.00Ca** | **1.43** | **0.12** |
| **2.00Ca** | **1.36** | **0.15** |
| **3.50Ca** | **1.29** | **0.11** |

### Example 32

Example 27 was repeated using a dressing made using a cross-linked copolymer of NaAMPS and SPAK according to the following details:
Pre-gel: 70 parts by weight of 58% aqueous solution of the sodium salt of acrylamidomethylpropanesulphonic acid (Na AMPS, LZ2405 Lubrizol), 30 parts glycerol, and 0.14 parts of a 1 to 10 (by weight) mixture of Daracure 1173 photoinitiator (Ciba Speciality Chemicals) and IRR280 cross-linker (PEG400 diacrylate, UCB Chemicals).

The results (mean and standard deviation (SD)) of the ion analysis from the top and bottom filter papers are shown in the Table below, units mmol/l measured at time = 0, 0.5 hours, 1.00 hours, 2.00 hours and 3.50 hours:

| **Time (hours)** | **Mean** | **SD** |
|---|---|---|
| **0.00 Sodium top** | **144.00** | |
| **0.50Na** | **230.00** | **0.00** |
| **1.00Na** | **230.00** | **0.00** |
| **2.00Na** | **230.00** | **0.00** |
| **3.50Na** | **230.00** | **0.00** |
| | | |
| **0.00 Potassium top** | **5.95** | |
| **0.50K** | **3.43** | **0.12** |
| **1.00K** | **3.30** | **0.26** |
| **2.00K** | **3.33** | **0.32** |
| **3.50K** | **3.27** | **0.21** |
| | | |
| **0.00 Calcium top** | **1.80** | |
| **0.50Ca** | **1.52** | **0.15** |
| **1.00Ca** | **1.47** | **0.07** |
| **2.00Ca** | **1.46** | **0.15** |
| **3.50Ca** | **1.15** | **0.12** |
| | | |
| **0.00 Sodium bottom** | **144.00** | |
| **0.50Na** | **155.77** | **8.91** |
| **1.00Na** | **168.27** | **8.81** |
| **2.00Na** | **199.63** | **16.61** |
| **3.50Na** | **201.07** | **25.33** |
| | | |
| **0.00 Potassium bottom** | **5.92** | |
| **0.50K** | **5.87** | **0.23** |
| **1.00K** | **6.00** | **0.17** |
| **2.00K** | **6.30** | **0.35** |
| **3.50K** | **5.33** | **0.47** |
| | | |
| **0.00 Calcium bottom** | **1.72** | |
| **0.50Ca** | **1.73** | **0.13** |
| **1.00Ca** | **1.65** | **0.28** |
| **2.00Ca** | **1.61** | **0.22** |
| **3.50Ca** | **1.45** | **0.15** |

### Example 33

The experiments of Examples 27 to 32 show that ion and osmotic gradients are established in a model wound system adjacent a hydrogel dressing according to the present disclosure.

In Example 33, the osmolarity of the solutions obtained from the top and bottom filter papers in Examples 27 to 32 was measured using the method described in Example 16.

The results show that the osmolarity gradient is at least 50 milliosmoles per litre for non-glycerol-containing hydrogels over the period of 0.5 to 3.5 hours after start, and is not less than 300 milliosmoles per litre for glycerol-containing hydrogels. We expect that this osmolarity of not less than about 300 milliosmoles per litre will be found in all hydrogel compositions according to the present disclosure having a substantial glycerol (organic plasticiser) content, e.g. greater than about 5 parts by weight per hundred parts by weight of other ingredients of the composition.

### Examples 34 to 48 and Reference Examples 49 and 50 - Further Hydrogel Compositions

Examples 34 to 47 illustrate further suitable hydrogel compositions which may be used with suitable sheet support members as described herein to provide a dressing for use in the present invention.

The curing conditions used in these Examples was as stated above in relation to Examples 1 to 15.

### Example 34

Pre-gel: 70 parts by weight of 58% aqueous solution of the sodium salt of acrylamidomethylpropanesulphonic acid (NaAMPS, LZ2405 Lubrizol), 30 parts glycerol, 3 parts acrylic acid (3-sulphopropyl) ester potassium salt, commonly known as SPA or SPAK (SPA or SPAK is available commercially in the form of a pure solid from Raschig and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 35

Pre-gel: 70 parts by weight of 58% aqueous solution of the sodium salt of acrylamidomethylpropanesulphonic acid (NaAMPS, LZ2405 Lubrizol), 30 parts glycerol, 3 parts acrylic acid (3-sulphopropyl) ester potassium salt, commonly known as SPA or SPAK (SPA or SPAK is available commercially in the form of a pure solid from Raschig and 0.09 parts of a 1 to 10 (by weight) mixture of Daracure 1173 photoinitiator (Ciba Speciality Chemicals) and IRR280 cross-linker (PEG400 diacrylate, UCB Chemicals).

### Example 36

Pre-gel: 70 parts by weight of 58% aqueous solution of the sodium salt of acrylamidomethylpropanesulphonic acid (NaAMPS, LZ2405 Lubrizol), 30 parts glycerol, 3 parts acrylic acid (3-sulphopropyl) ester potassium salt, commonly known as SPA or SPAK (SPA or SPAK is available commercially in the form of a pure solid from Raschig and 0.2 parts of a 1 to 10 (by weight) mixture of Daracure 1173 photoinitiator (Ciba Speciality Chemicals) and IRR280 cross-linker (PEG400 diacrylate, UCB Chemicals).

### Example 37

Pre-gel: 70 parts by weight of 58% aqueous solution of the sodium salt of acrylamidomethylpropanesulphonic acid (NaAMPS, LZ2405 Lubrizol), 30 parts glycerol, 3 parts acrylic acid (3-sulphopropyl) ester Sodium salt in the form of a pure solid from Raschig and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 38

Pre-gel: 70 parts by weight of 58% aqueous solution of the sodium salt of acrylamidomethylpropanesulphonic acid (NaAMPS, LZ2405 Lubrizol), 30 parts glycerol, 0.1 parts acrylic acid (3-sulphopropyl) ester Sodium salt in the form of a pure solid from Raschig and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 39

Pre-gel: 70 parts by weight of 58% aqueous solution of the sodium salt of acrylamidomethylpropanesulphonic acid (NaAMPS, LZ2405 Lubrizol), 30 parts glycerol, 0.1 parts acrylic acid (3-sulphopropyl) ester potassium salt, commonly known as SPA or SPAK (SPA or SPAK is available commercially in the form of a pure solid from Raschig and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 40

Pre-gel: 70 parts by weight of 58% aqueous solution of the sodium salt of acrylamidomethylpropanesulphonic acid (NaAMPS, LZ2405 Lubrizol), 30 parts glycerol, 0.05 parts acrylic acid (3-sulphopropyl) ester potassium salt, commonly known as SPA or SPAK (SPA or SPAK is available commercially in the form of a pure solid from Raschig and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 41

Pre-gel: 70 parts by weight of 58% aqueous solution of the sodium salt of acrylamidomethylpropanesulphonic acid (NaAMPS, LZ2405 Lubrizol), 30 parts glycerol, 0.1 parts glucose (Aldrich) 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 42

Pre-gel: 70 parts by weight of 58% aqueous solution of the sodium salt of acrylamidomethylpropanesulphonic acid (NaAMPS, LZ2405 Lubrizol), 30 parts glycerol, 10 parts glucose (Aldrich) 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 43

Pre-gel: 60 parts by weight of 58% aqueous solution of the sodium salt of acrylamidomethylpropanesulphonic acid (NaAMPS, LZ2405 Lubrizol), 10 parts glycerol, 30 parts water, 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 44

Pre-gel: 70 parts by weight of 58% aqueous solution of the sodium salt of acrylamidomethylpropanesulphonic acid (NaAMPS, LZ2405 Lubrizol), 30 parts glycerol, 0.05 parts Ammonium AMPS (Lubrizol) and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 45

Pre-gel: 70 parts by weight of 58% aqueous solution of the sodium salt of acrylamidomethylpropanesulphonic acid (NaAMPS, LZ2405 Lubrizol), 30 parts glycerol, 1 parts Ammonium AMPS (Lubrizol) and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 46

Pre-gel: 70 parts by weight of 58% aqueous solution of the sodium salt of acrylamidomethylpropanesulphonic acid (NaAMPS, LZ2405 Lubrizol), 30 parts glycerol, 0.05 parts Potassium AMPS (Lubrizol) and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 47

Pre-gel: 70 parts by weight of 58% aqueous solution of the sodium salt of acrylamidomethylpropanesulphonic acid (NaAMPS, LZ2405 Lubrizol), 30 parts glycerol, 1 parts Potassium AMPS (Lubrizol) and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Example 48

Pre-gel: 35 parts by weight of SPDA (N,N-dimethyl-N-(2-acryloyloxyethyl)-N-(3-sulphopropyl)amonium betaine), a sulfobetaine monomer from Raschig, 30 parts water, 2 parts of 58% aqueous solution of the sodium salt of acrylamidomethylpropanesulphonic acid (NaAMPS, LZ2405 Lubrizol), 30 parts glycerol, and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Reference Example 49

Pre-gel: 35 parts by weight of SPDA (N,N-dimethyl-N-(2-acryloyloxyethyl)-N-(3-sulphopropyl)amonium betaine), a sulfobetaine monomer from Raschig, 30 parts water, 10 parts of 58% aqueous solution of the sodium salt of acrylamidomethylpropanesulphonic acid (NaAMPS, LZ2405 Lubrizol), 30 parts glycerol, 1 parts Potassium AMPS (Lubrizol) 30 parts glycerol, and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Reference Example 50

Pre-gel: 35 parts by weight of hydroxyethylacrylamide monomer from Raschig 30 parts water, 2 parts of 58% aqueous solution of the sodium salt of acrylamidomethylpropanesulphonic acid (NaAMPS, LZ2405 Lubrizol), 30 parts glycerol, 1 parts Potassium AMPS (Lubrizol) 30 parts glycerol, and 0.14 parts of a 1 to 10 (by weight) mixture of Daracure 1173 photoinitiator (Ciba Speciality Chemicals) and IRR280 cross-linker (PEG400 diacrylate, UCB Chemicals).

### Industrial applicability

The present invention provides an effective method of microbial killing, useful in the treatment chronic ulcerated skin lesions (e.g. chronic venous or arterial leg ulcers) to promote their healing.

In the context of the treatment of wounds, the method makes available simultaneous reduction of one or more undesirable characteristics of a chronic skin lesion, selected from pain associated with the wound, pain associated with changing of the dressing, exudation, malodour, irritation and hyperkeratosis.

Undesirable effects of conventional dressings for chronic skin lesions, for example maceration, incomplete absorption of exudate, excoriation, scarring of the final healed tissue, contact dermatitis, varicose eczema or skin stripping can be reduced using the present invention in the context of wound treatment.

The hydrogel (dressing) used in the present invention is easy to apply and change, with resultant cost savings and efficiency enhancements.

Without wishing to be bound by theory, the hydrogel dressing is believed to mimic the natural extracellular matrix of a normal healing wound, and in particular certain sulphonated proteoglycans of the extracellular matrix such as heparin, using a moist wound healing environment where, in contrast to prior methods, the water levels are controlled to avoid the disadvantages of too much or too little moisture. The sulphonyl groups are believed to hold a relatively large hydration shell around them in the hydrogel, which may contribute to the very substantial wound healing and antimicrobial effects found with the hydrogels of the present invention.

The above broadly describes the present invention, without limitation. Variations and modifications as will be readily apparent to those of ordinary skill in this art are intended to be covered by this application and all subsequent patents.

## Claims

1. A topical hydrogel composition comprising a hydrophilic polymer carrying multiple pendant sulphonyl groups on each polymer molecule, for use in the treatment of a chronic ulcerous skin lesion, in a human or non-human mammal, particularly a human wherein the sulfonyl groups contain sulpho groups in salt form,
wherein the salt form is sodium alone or a combination of sodium and potassium counterions, and the molar ratio of sodium ions to potassium ions in the hydrogel composition is in the range of about 100:0 to 100:10.

2. A topical hydrogel composition for use according to claim 1, wherein the molar ratio of sodium ions to potassium ions in the hydrogel composition is in the range of about 100:0.1 to 100:5.

3. A topical hydrogel composition for use according to claim 1, wherein the molar ratio of sodium ions to potassium ions in the hydrogel composition is in the range of about 100:1 and about 100:5.

4. A topical hydrogel composition for use according to any one of the preceding claims, wherein the polymer is formed from monomers selected from the sodium salt of 2-acrylamido-2-methylpropane sulphonic acid and acrylic acid (3-sulphopropyl) ester potassium salt.

5. A topical hydrogel composition for use according to claim 1, wherein the hydrogel additionally comprises glycerol.

6. A topical hydrogel composition for use according to any one of the preceding claims, wherein the hydrogel composition is prepared by free radical polymerisation with cross-linking and wherein the photoinitiator is 2-hydroxy-2-propyl phenyl ketone.

7. A topical hydrogel composition for use according to claim 1, wherein the hydrogel composition additionally comprises polymers selected from polyoxypropyelene-polyoxyethylene block copolymer or poly-(methyl vinyl ether alt maleic anhydride).

8. A topical hydrogel composition for use according to any one of the preceding claims, wherein the chronic ulcerous skin lesion is a venous leg ulcer, venous foot ulcer, arterial leg ulcer, decubitus ulcer, post-surgical ulcerous lesions and chronic burn lesions.

9. A topical hydrogel composition for use according to claim 1, wherein the hydrogel composition is free of any styrene sulphonate component.

10. A topical hydrogel composition for use according to claim 1, wherein the hydrogel composition contacts the wound for an effective period of time, the effective period of time being between 3 days and 6 weeks.

11. A topical hydrogel composition for use according to claim 1, wherein the hydrophilic polymer additionally carries multiple pendant carboxylic groups on each polymer molecule.

## Patentansprüche

1. Topische Hydrogelzusammensetzung, umfassend ein hydrophiles Polymer mit mehreren Sulfonylseitengruppen an jedem Polymermolekül, die bei der Behandlung einer chronischen geschwürigen Hautläsion bei einem menschlichen oder nichtmenschlichen Säuger, insbesondere aber bei einem Menschen eingesetzt wird, wobei die Sulfonylgruppen salzförmige Sulfogruppen aufweisen,
wobei die Salzform nur aus Natrium oder aus einer Kombination von Natrium- und Kaliumgegenionen besteht und in der Hydrogelzusammensetzung das Molverhältnis von Natriumionen zu Kaliumionen etwa zwischen 100:0 und 100:10 liegt.

2. Topische Hydrogelzusammensetzung zur Verwendung gemäß Anspruch 1, wobei in der Hydrogelzusammensetzung das Molverhältnis von Natriumionen zu Kaliumionen etwa zwischen 100:0,1 und 100:5 liegt.

3. Topische Hydrogelzusammensetzung zur Verwendung gemäß Anspruch 1, wobei in der Hydrogelzusammensetzung das Molverhältnis von Natriumionen zu Kaliumionen etwa zwischen 100:1 und 100:5 liegt.

4. Topische Hydrogelzusammensetzung zur Verwendung gemäß einem der vorhergehenden Ansprüche,
wobei das Polymer aus Monomeren gebildet wird, die aus dem Natriumsalz der 2-Acrylamido-2-Methylpropansulfonsäure und dem Acrylsäure- (3-Sulfopropyl) Ester-Kalisalz ausgewählt wurden.

5. Topische Hydrogelzusammensetzung zur Verwendung gemäß Anspruch 1, wobei das Hydrogel zusätzlich Glycerin enthält.

6. Topische Hydrogelzusammensetzung zur Verwendung gemäß einem der vorhergehenden Ansprüche,
wobei die Hydrogelzusammensetzung durch Radikalkettenpolymerisation mit Vernetzung hergestellt wird und wobei es sich bei dem Photoinitiator um 2-Hydroxy-2-Propylphenylketon handelt.

7. Topische Hydrogelzusammensetzung zur Verwendung gemäß Anspruch 1, wobei die Hydrogelzusammensetzung zusätzlich Polymere umfasst, bei denen es sich um Polyoxypropylen-Polyoxyethylen-Blockcopolymer oder Polymethylvinylethersalz-Maleinsäureanhydrid handelt.

8. Topische Hydrogelzusammensetzung zur Verwendung gemäß einem der vorhergehenden Ansprüche,
wobei es sich bei der chronischen geschwürigen Hautläsion um ein venös verursachtes Beingeschwür, ein venös verursachtes Fußgeschwür, ein arteriell verursachtes Beingeschwür, einen Dekubitus, postoperative geschwürige Läsionen und/oder chronische Brandläsionen handelt.

9. Topische Hydrogelzusammensetzung zur Verwendung gemäß Anspruch 1, wobei die Hydrogelzusammensetzung keine Styrolsulfonatkomponenten enthält.

10. Topische Hydrogelzusammensetzung zur Verwendung gemäß Anspruch 1, wobei die Hydrogelzusammensetzung die Wunde während der Anwendungsdauer berührt, wobei die Anwendungsdauer zwischen 3 Tagen und 6 Wochen liegt.

11. Topische Hydrogelzusammensetzung zur Verwendung gemäß Anspruch 1, wobei das hydrophile Polymer an jedem Polymermolekül zusätzlich mehrere Carboxylseitengruppen aufweist.

## Revendications

1. Une composition d'hydrogel topique comprenant un polymère hydrophile transportant plusieurs groupes sulfonyle sur chaque molécule de polymère, destinée à être utilisée dans le traitement d'une lésion cutanée ulcéreuse chronique, chez un humain ou un mammifère non humain, particulièrement un humain dans laquelle les groupes sulfonyle contiennent des groupes sulfo en forme de sel, dans laquelle la forme de sel est le sodium seul ou une combinaison de contreions de sodium et de potassium et le rapport molaire des ions sodium aux ions de potassium dans la composition d'hydrogel est dans la plage allant d'environ 100:0 à 100:10.

2. Une composition d'hydrogel topique destinée à être utilisée selon la revendication 1, dans laquelle le rapport molaire des ions sodium aux ions potassium dans la composition de l'hydrogel est dans la plage allant d'environ 100:0,1 à 100:5.

3. Une composition d'hydrogel topique destinée à être utilisée selon la revendication 1, dans laquelle le rapport molaire des ions sodium aux ions de potassium dans la composition d'hydrogel est dans la plage allant d'environ 100:1 à 100:5.

4. Une composition d'hydrogel topique destinée à être utilisée selon l'une quelconque des revendications précédentes, dans laquelle le polymère est formé à partir des monomères sélectionnés parmi le sel sodique de l'acide 2-acrylamido 2-méthylpropane sulfonique et le sel de potassium de l'ester d'acide (3-sulphopropyl) acrylique.

5. Une composition d'hydrogel topique destinée à être utilisée selon la revendication 1, dans laquelle l'hydrogel comporte en plus le glycérol.

6. Une composition d'hydrogel topique destinée à être utilisée selon l'une quelconque des revendications précédentes, dans laquelle la composition d'hydrogel est préparée par polymérisation de radicaux libres avec réticulation et dans laquelle le photoinitiateur est 2-hydroxy-2-propyl-phényl cétone.

7. Une composition d'hydrogel topique destinée à être utilisée selon la revendication 1, dans laquelle la composition d'hydrogel comporte en outre des polymères sélectionnés parmi le copolymère à blocs polyoxypropyélène-polyoxyéthylène ou l'anhydride maléique poly-(méthyle-vinyle-éther-alt).

8. Une composition d'hydrogel topique destinée à être utilisée selon l'une quelconque des revendications précédentes, dans laquelle la lésion cutanée ulcéreuse chronique est un ulcère veineux de la jambe, un ulcère veineux du pied, un ulcère artériel de la jambe, un ulcère de décubitus, des lésions ulcéreuses post-chirurgicales et des lésions de brulure chroniques.

9. Une composition d'hydrogel topique destinée à être utilisée selon la revendication 1, dans laquelle la composition d'hydrogel est exempte de tout composant de sulphonate de styrène.

10. Une composition d'hydrogel topique destinée à être utilisée selon la revendication 1, dans laquelle la composition d'hydrogel entre en contact avec la blessure pendant une période de temps effective, la période de temps effective étant entre 3 jours et 6 semaines.

11. Une composition d'hydrogel topique destinée à être utilisée selon la revendication 1, dans laquelle le polymère hydrophile transporte en outre plusieurs groupes carboxyliques sur chaque molécule de polymère.
